(19) Europäisches Patentamt
European Patent Office
Office européen des brevets

(11) EP 2 649 193 B1

(12) EUROPEAN PATENT SPECIFICATION

(45) Date of publication and mention
of the grant of the patent:
**17.06.2020 Bulletin 2020/25**

(51) Int Cl.:
*C12Q 1/02* *(2006.01)*      *C12Q 1/06* *(2006.01)*
*C12M 1/16* *(2006.01)*      *C12M 1/34* *(2006.01)*
*G01N 1/40* *(2006.01)*      *C12M 1/02* *(2006.01)*
*C12Q 1/04* *(2006.01)*      *G01N 33/84* *(2006.01)*
*C12M 1/00* *(2006.01)*

(21) Application number: **11847458.4**

(22) Date of filing: **30.11.2011**

(86) International application number:
**PCT/US2011/062618**

(87) International publication number:
**WO 2012/078426 (14.06.2012 Gazette 2012/24)**

(54) **MICROORGANISM CONCENTRATION PROCESS AND DEVICE**

VERFAHREN UND VORRICHTUNG ZUR KONZENTRATION VON MIKROORGANISMEN

PROCÉDÉ ET DISPOSITIF DE CONCENTRATION DE MICROORGANISMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2010 US 420119 P**

(43) Date of publication of application:
**16.10.2013 Bulletin 2013/42**

(73) Proprietor: **3M Innovative Properties Company**
**St. Paul, MN 55133-3427 (US)**

(72) Inventors:
• **KSHIRSAGAR, Manjiri T.**
**Saint Paul**
**Minnesota 55133-3427 (US)**
• **RABINS, Andrew W.**
**Saint Paul**
**Minnesota 55133-3427 (US)**

(74) Representative: **Mathys & Squire**
**Mathys & Squire Europe LLP**
**Theatinerstraße 7**
**80333 München (DE)**

(56) References cited:
EP-A2- 0 443 853          WO-A1-93/01494
WO-A1-93/06924           WO-A1-98/02224
WO-A1-99/27387           WO-A1-2010/114725
WO-A1-2010/114727        WO-A1-2010/114727
WO-A2-2010/078399        US-A- 5 498 478
US-A- 5 637 506          US-A1- 2004 159 605
US-A1- 2008 164 214      US-A1- 2010 190 171

**Description**

**FIELD**

This invention relates to processes for capturing or concentrating microorganisms such that they remain viable for detection or assay. In other aspects, this invention also relates to concentration devices (and diagnostic kits comprising the devices) for use in carrying out such processes and to methods for device preparation.

**BACKGROUND**

WO 93/06924 discloses an article that is useful in separation science, specifically for extraction, purification, or removal of soluble or insoluble organic or inorganic materials from fluids including water, wastewater, and air, wherein the article is a particle loaded, porous, fibrous, at least one of compressed or fused, article comprising a) a nonwoven fibrous polymeric web, and b) sorptive particles enmeshed in said web, wherein said particle loaded fibrous article has a Gurley number of at least two seconds.

WO 98/02224 discloses a method for the concentration and quantification of a targeted ion from a source solution which comprises a) bringing a measured volume of said source solution into contact with a porous web or membrane having enmeshed therein, in particulate form, an ion recognition substrate having an affinity for the targeted ion and thereby forming a complex between said targeted ion and said ion recognition substrate enmeshed in said web; b) removing said volume of said source solution from contact with said web; and c) determining the concentration of said targeted ion in said measured volume of source solution by means of colorimetric analysis of said targeted ion complexed to said ion recognition substrate enmeshed in said web.

Food-borne illnesses and hospital-acquired infections resulting from microorganism contamination are a concern in numerous locations all over the world. Thus, it is often desirable or necessary to assay for the presence of bacteria or other microorganisms in various clinical, food, environmental, or other samples, in order to determine the identity and/or the quantity of the microorganisms present.

Bacterial DNA or bacterial RNA, for example, can be assayed to assess the presence or absence of a particular bacterial species even in the presence of other bacterial species. The ability to detect the presence of a particular bacterium, however, depends, at least in part, on the concentration of the bacterium in the sample being analyzed. Bacterial samples can be plated or cultured to increase the numbers of the bacteria in the sample to ensure an adequate level for detection, but the culturing step often requires substantial time and therefore can significantly delay the assessment results.

Concentration of the bacteria in the sample can shorten the culturing time or even eliminate the need for a culturing step. Thus, methods have been developed to isolate (and thereby concentrate) particular bacterial strains by using antibodies specific to the strain (for example, in the form of antibody-coated magnetic or non-magnetic particles). Such methods, however, have tended to be expensive and still somewhat slower than desired for at least some diagnostic applications.

Concentration methods that are not strain-specific have also been used (for example, to obtain a more general assessment of the microorganisms present in a sample). After concentration of a mixed population of microorganisms, the presence of particular strains can be determined, if desired, by using strain-specific probes.

Non-specific concentration or capture of microorganisms has been achieved through methods based upon carbohydrate and lectin protein interactions. Chitosan-coated supports have been used as non-specific capture devices, and substances (for example, carbohydrates, vitamins, iron-chelating compounds, and siderophores) that serve as nutrients for microorganisms have also been described as being useful as ligands to provide non-specific capture of microorganisms.

Various inorganic materials (for example, hydroxyapatite and metal hydroxides) have been used to non-specifically bind and concentrate bacteria. Physical concentration methods (for example, filtration, chromatography, centrifugation, and gravitational settling) have also been utilized for non-specific capture, with and/or without the use of inorganic binding agents. Such non-specific concentration methods have varied in speed (at least some food testing procedures still requiring at least overnight incubation as a primary cultural enrichment step), cost (at least some requiring expensive equipment, materials, and/or trained technicians), sample requirements (for example, sample nature and/or volume limitations), space requirements, ease of use (at least some requiring complicated multi-step processes), suitability for on-site use, and/or effectiveness.

WO 2010/114727 discloses a process comprises (a) providing a concentration device comprising a sintered porous polymer matrix comprising at least one concentration agent that comprises an amorphous metal silicate and that has a surface composition having a metal atom to silicon atom ratio of less than or equal to 0.5, as determined by X-ray photoelectron spectroscopy (XPS); (b) providing a sample (in particular, in the form of a fluid) comprising at least one microorganism strain; and (c) contacting the concentration device with the sample (in particular, by passing the sample at least once through the concentration device (for example by pumping)) such that at least a portion of the at least one microorganism strain is bound to or captured by the concentration device. In the Examples, contacting is effected by passing a sample through the concentration device by pumping.

**SUMMARY**

Thus, we recognize that there is an urgent need for processes for rapidly detecting pathogenic microorganisms. Such processes will preferably be not only rapid but also low in cost, simple (involving no complex equipment or procedures), and/or effective under a variety of conditions (for example, with varying types of sample matrices and/or pathogenic microorganisms, varying microorganism loads, and varying sample volumes).

Briefly, in one aspect, this invention provides a process for non-specifically concentrating the strains of microorganisms (for example, strains of bacteria, fungi, yeasts, protozoans, viruses (including both non-enveloped and enveloped viruses), and bacterial endospores) present in a sample, such that the microorganisms remain viable for the purpose of detection or assay of one or more of the strains. The process comprises (a) providing a concentration device comprising (1) a porous fibrous nonwoven matrix, and (2) a plurality of particles of at least one concentration agent that comprises an amorphous metal silicate having a surface composition having a metal atom to silicon atom ratio of less than or equal to 0.5, as determined by X-ray photoelectron spectroscopy (XPS), the particles being enmeshed in the porous fibrous nonwoven matrix; (b) providing a sample (preferably, in the form of a fluid) comprising at least one target cellular analyte, wherein the target analyte is a cell or a micro-organism (for example, at least one microorganism strain); (c) contacting the concentration device with the sample (preferably, by passing the sample through the concentration device) such that at least a portion of the at least one target cellular analyte is bound to or captured by the concentration device; and (d) detecting the presence of at least one bound target cellular analyte.

The process can optionally further comprise separating the concentration device from the sample and/or culturally enriching at least one bound target cellular analyte (for example, by incubating the separated concentration device in a general or microorganism-specific culture medium, depending upon whether general or selective microorganism enrichment is desired) and/or isolating or separating captured target cellular analytes (for example, microorganisms or one or more components thereof) from the concentration device after sample contacting (for example, by passing an elution agent or a lysis agent through the concentration device). If desired, however, detection of the target cellular analyte (for example, by culture-based, microscopy/imaging, genetic, luminescence-based, or immunologic detection methods) generally can be carried out in the presence of the concentration device.

The process of the invention does not target a specific cellular analyte (for example, a particular microorganism strain). Rather, it has been discovered that a concentration device comprising certain relatively inexpensive, inorganic materials enmeshed in a porous fibrous nonwoven matrix can be surprisingly effective in capturing a variety of microorganisms (and surprisingly effective in isolating or separating the captured microorganisms via elution, relative to corresponding devices without the inorganic material). Such devices can be used to concentrate the microorganism strains present in a sample (for example, a food sample) in a non-strain-specific manner, so that one or more of the microorganism strains (preferably, one or more strains of bacteria) can be more easily and rapidly assayed.

The process of the invention is relatively simple and low in cost (requiring no complex equipment or expensive strain-specific materials) and can be relatively fast (preferred embodiments capturing at least about 70 percent (more preferably, at least about 80 percent; most preferably, at least about 90 percent) of the microorganisms present in a relatively homogeneous fluid sample in less than about 10 minutes, relative to a corresponding control sample having no contact with the concentration device). In contrast with the use of particulate concentration agents alone, the process can be surprisingly effective in microorganism capture with only relatively short sample contact times (for example, as short as about 20 seconds) and without the need for a settling step.

The process of the invention is also surprisingly "assay-friendly." Detection can generally be effected in the presence of the concentration device without significant assay interference (for example, without detection errors resulting from the absorption of assay reagents by the concentration device or resulting from the leaching of assay inhibitors from the concentration device). This enables concentration and detection to be carried out quickly (for example, as quickly as 10 minutes or less) in the sampling environment.

In addition, the process can be effective with a variety of microorganisms (including pathogens such as both gram positive and gram negative bacteria) and with a variety of samples (different sample matrices and, unlike at least some prior art methods, even samples having low microorganism content and/or large volumes). Thus, at least some embodiments of the process of the invention can meet the above-cited urgent need for low-cost, simple processes for rapidly detecting pathogenic microorganisms under a variety of conditions.

The process of the invention can be especially advantageous for concentrating the microorganisms in food samples (for example, particulate-containing food samples, especially those comprising relatively coarse particulates), as the concentration device used in the process can exhibit at least somewhat greater resistance to clogging than at least some filtration devices such as absolute micron filters. This can facilitate more complete sample processing (which is essential to eliminating false negative assays in food testing) and the handling of relatively large volume samples (for example, under field conditions).

A preferred concentration process comprises

(a) providing a concentration device comprising

(1) a porous fibrous nonwoven matrix comprising (i) at least one fibrillated fiber and (ii) at least one polymeric binder, and
(2) a plurality of particles of at least one concentration agent that comprises at least one amorphous, spheroidized metal silicate having a surface composition having a metal atom to silicon atom ratio of less than or equal to 0.5, as determined by X-ray photoelectron spectroscopy (XPS);

the particles being enmeshed in the porous fibrous nonwoven matrix;
(b) providing a fluid sample comprising at least one target cellular analyte, wherein the target analyte is a cell or a micro-organism and
(c) passing the fluid sample through the concentration device in a manner such that at least a portion of the at least one target cellular analyte, wherein the target analyte is a cell or a micro-organism, is bound to or captured by the concentration device.

In another aspect, the invention also provides a concentration device comprising (a) a porous fibrous nonwoven matrix comprising polymer fibers and inorganic fibers; and (b) a plurality of particles of at least one concentration agent that comprises an amorphous, spheroidized metal silicate having a surface composition having a metal atom to silicon atom ratio of less than or equal to 0.5, as determined by X-ray photoelectron spectroscopy (XPS); wherein the particles are enmeshed in the porous fibrous nonwoven matrix. The invention also provides a diagnostic kit for use in carrying out the concentration process of the invention, the kit comprising (a) at least one concentration device of the invention; and (b) at least one testing container or testing reagent for use in carrying out the above-described concentration process.

In yet another aspect, the invention provides a process for preparing a concentration device adapted to concentrate a target cellular analyte, wherein the target analyte is a cell or a micro-organism comprising (a) providing a plurality of fibers comprising polymer fibers and inorganic fibers; (b) providing a plurality of particles of at least one concentration agent that comprises an amorphous, spheroidized metal silicate having a surface composition having a metal atom to silicon atom ratio of less than or equal to 0.5, as determined by X-ray photoelectron spectroscopy (XPS); and (c) forming at least a portion of the plurality of fibers into a porous fibrous nonwoven matrix having at least a portion of the plurality of particles enmeshed therein.

## DETAILED DESCRIPTION

In the following detailed description, various sets of numerical ranges (for example, of the number of carbon atoms in a particular moiety, of the amount of a particular component) are described, and, within each set, any lower limit of a range can be paired with any upper limit of a range. Such numerical ranges also are meant to include all numbers subsumed within the range (for example, 1 to 5 includes 1, 1.5, 2, 2.75, 3, 3.80, 4, 5, and so forth).

As used herein, the term "and/or" means one or all of the listed elements or a combination of any two or more of the listed elements.

The words "preferred" and "preferably" refer to embodiments of the invention that may afford certain benefits under certain circumstances. Other embodiments may also be preferred, however, under the same or other circumstances. Furthermore, the recitation of one or more preferred embodiments does not imply that other embodiments are not useful, and is not intended to exclude other embodiments from the scope of the invention.

The term "comprises" and variations thereof do not have a limiting meaning where these terms appear in the description and claims.

As used herein, "a," "an," "the," "at least one," and "one or more" are used interchangeably.

The above "Summary of the Invention" section is not intended to describe every embodiment or every implementation of the invention. The detailed description that follows more particularly describes illustrative embodiments. Throughout the detailed description, guidance is provided through lists of examples, which examples can be used in various combinations. In each instance, a recited list serves only as a representative group and should not be interpreted as being an exclusive list.

## Definitions

As used in this patent application:

"aramid" means an aromatic polyamide;

"cellular analyte" means an analyte of cellular origin (that is, a microorganism or a component thereof (for example, a cell or a cellular component such as deoxyribonucleic acid (DNA) or ribonucleic acid (RNA), proteins, nucleotides such as adenosine triphosphate (ATP), and combinations thereof); references to a microorganism or microorganism strain throughout this specification are meant to apply more generally to any cellular analyte);

"concentration agent" means a material or composition that binds cellular analytes (preferably, having a cellular analyte capture or binding efficiency of at least about 60 percent; more preferably, at least about 70 percent; even more preferably, at least about 80 percent; most preferably, at least about 90 percent);

"culture device" means a device that can be used to propagate microorganisms under conditions that will permit at least one cell division to occur (preferably, culture devices include a housing to reduce or minimize the possibility of incidental contamination and/or a source of nutrients to support the growth of microorganisms);

"detection" means the identification of a cellular analyte (for example, at least a component of a target microorganism, which thereby determines that the target microorganism is present);

"enmeshed" (in regard to particles of concentration agent in a fibrous nonwoven matrix) means that the particles are entrapped in the fibrous nonwoven matrix (and, preferably, distributed within it), rather than merely being borne on its surface;

"fibrillated" (in regard to fibers or fibrous material) means treated (for example, by beating) in a manner that forms fibrils or branches attached to a fiber's main trunk;

"fibrous nonwoven matrix" means a web or medium, other than a woven or knitted fabric, comprising interlaid fibers (for example, a web comprising fibers that are interlaid by meltblowing, spunbonding, or other air laying techniques; carding; wet laying;);

"genetic detection" means the identification of a component of genetic material such as DNA or RNA that is derived from a target microorganism;

"immunologic detection" means the identification of an antigenic material such as a protein or a proteoglycan that is derived from a target microorganism;

"microorganism" means any cell or particle having genetic material suitable for analysis or detection (including, for example, bacteria, yeasts, viruses, and bacterial endospores);

"microorganism strain" means a particular type of microorganism that is distinguishable through a detection method (for example, microorganisms of different genera, of different species within a genera, or of different isolates within a species);

"para-aramid" means an aromatic polyamide having its amide linkages bonded to substituted (for example, alkyl-substituted) or unsubstituted benzene rings in para-relation (bonded to carbon numbers one and four);

"sample" means a substance or material that is collected (for example, to be analyzed);

"sample matrix" means the components of a sample other than cellular analytes;

"target cellular analyte" means any cellular analyte that is desired to be detected;

"target microorganism" means any microorganism that is desired to be detected; and

"through pore" (in reference to a porous matrix) means a pore that comprises a passageway or channel (with separate inlet and outlet) through the matrix.

## Concentration Agent

Concentration agents suitable for use in carrying out the process of the invention include those particulate concentration agents that comprise at least one metal silicate. The metal silicates are amorphous.

Concentration or capture using such concentration agents is generally not specific to any particular strain, species, or type of microorganism and therefore provides for the concentration of a general population of microorganisms in a sample. Specific strains of microorganisms can then be detected from among the captured microorganism population using any known detection method with strain-specific probes. Thus, the concentration agents can be used for the detection of microbial contaminants or pathogens (particularly food-borne pathogens such as bacteria) in clinical, food, environmental, or other samples.

When dispersed or suspended in water systems, inorganic materials such as metal silicates exhibit surface charges that are characteristic of the material and the pH of the water system. The potential across the material-water interface is called the "zeta potential," which can be calculated from electrophoretic mobilities (that is, from the rates at which the particles of material travel between charged electrodes placed in the water system). Preferably, the concentration agents have a negative zeta potential at a pH of about 7.

Useful metal silicates include silicates of metals such as magnesium, calcium, zinc, aluminum, iron, titanium (preferably, magnesium, zinc, iron, and titanium; more preferably, magnesium), and combinations thereof. Preferred are amorphous metal silicates in at least partially fused particulate form (more preferably, amorphous, spheroidized metal silicates; most preferably, amorphous, spheroidized magnesium silicate). Metal silicates are known and can be chemically synthesized by known methods or obtained through the mining and processing of raw ores that are naturally-occurring.

Amorphous, at least partially fused particulate forms of metal silicates can be prepared by any of the known methods of melting or softening relatively small feed particles (for example, average particle sizes up to about 25 microns) under controlled conditions to make generally ellipsoidal or spheroidal particles (that is, particles having magnified two-dimensional images that are generally rounded and free of sharp corners or edges, including truly or substantially circular and elliptical shapes and any other rounded or curved shapes). Such methods include atomization, fire polishing, direct fusion. A preferred method is flame fusion, in which at least partially fused, substantially glassy particles are formed by direct fusion or fire polishing of solid feed particles (for example, as in the method described in U.S. Patent No. 6,045,913 (Castle)). Most preferably, such methods can be utilized to produce amorphous, spheroidized metal silicates by converting a substantial portion of irregularly-shaped feed particles (for example, from about 15 to about 99 volume percent; preferably, from about 50 to about 99 volume percent; more preferably, from about 75 to about 99 volume percent; most preferably, from about 90 to about 99 volume percent) to generally ellipsoidal or spheroidal particles.

Some amorphous metal silicates are commercially available. For example, amorphous, spheroidized magnesium silicate is commercially available for use in cosmetic formulations (for example, as 3M™ Cosmetic Microspheres CM-111, available from 3M Company, St. Paul, MN).

In addition to metal silicates, the concentration agents can further comprise other materials including oxides of metals (for example, iron or titanium), other crystalline materials, and combinations thereof. The concentration agents, however, for at least some applications, preferably contain essentially no crystalline silica.

In carrying out the process of the invention, the concentration agents can be used in essentially any particulate form (preferably, a relatively dry or volatiles-free form) that is amenable to blending with fibers to form the concentration device used in the process. For example, the concentration agents can be used in powder form or can be applied to a particulate support such as beads.

Preferably, the concentration agents are used in the form of a powder. Useful powders include those that comprise microparticles (preferably, microparticles having a particle size in the range of about 1 micrometer (more preferably, about 2 micrometers; even more preferably, about 3 micrometers; most preferably, about 4 micrometers) to about 100 micrometers (more preferably, about 50 micrometers; even more preferably, about 25 micrometers; most preferably, about 15 or 20 micrometers; where any lower limit can be paired with any upper limit of the range, as referenced above).

Concentration agents suitable for use in carrying out the process of the invention include those that comprise an amorphous metal silicate and that have a surface composition having a metal atom to silicon atom ratio of less than or equal to about 0.5 (preferably, less than or equal to about 0.4; more preferably, less than or equal to about 0.3; most preferably, less than or equal to about 0.2), as determined by X-ray photoelectron spectroscopy (XPS). Such concentration agents include those described in U.S. Patent Application Publication No. US 2010/0190171 published on July 29, 2010 (Kshirsagar et al.; 3M Innovative Properties Company).

Preferably, the surface composition of the particularly preferred concentration agents also comprises at least about 10 average atomic percent carbon (more preferably, at least about 12 average atomic percent carbon; most preferably, at least about 14 average atomic percent carbon), as determined by X-ray photoelectron spectroscopy (XPS). XPS is a technique that can determine the elemental composition of the outermost approximately 3 to 10 nanometers (nm) of a sample surface and that is sensitive to all elements in the periodic table except hydrogen and helium. XPS is a quantitative technique with detection limits for most elements in the 0.1 to 1 atomic percent concentration range. Surface composition assessment conditions for XPS include a take-off angle of 90 degrees measured with respect to the sample surface with a solid angle of acceptance of $\pm$ 10 degrees.

Such preferred metal silicate concentration agents can have zeta potentials that are more negative than that of, for example, a common metal silicate such as ordinary talc. Yet the concentration agents can be surprisingly more effective than talc in concentrating microorganisms such as bacteria, the surfaces of which generally tend to be negatively charged. Preferably, the concentration agents have a negative zeta potential at a pH of about 7 (more preferably, a Smoluchowski zeta potential in the range of about -9 millivolts to about -25 millivolts at a pH of about 7; even more preferably, a Smoluchowski zeta potential in the range of about -10 millivolts to about -20 millivolts at a pH of about 7; most preferably, a Smoluchowski zeta potential in the range of about -11 millivolts to about -15 millivolts at a pH of about 7).

Other particularly preferred concentration agents suitable for use in carrying out the process of the invention include those that comprise an adsorption buffer-modified, amorphous metal silicate. Such concentration agents include those described in U.S. Provisional Patent Application No. 61/289,213 filed on December 22, 2009 (Kshirsagar; 3M Innovative Properties Company).

## Concentration Device

Concentration devices suitable for use in carrying out the process of the invention include those that comprise (a) a porous fibrous nonwoven matrix comprising polymer fibers and inorganic fibers and (b) a plurality of particles of at least one concentration agent that comprises an amorphous, spheroidized metal silicate having a surface composition having a metal atom to silicon atom ratio of less than or equal to 0.5 (as described above), the particles being enmeshed in the porous fibrous nonwoven matrix. Such concentration devices can be prepared by essentially any process that is capable of providing a fibrous nonwoven matrix (that is, a web or medium, other than a woven or knitted fabric, comprising interlaid fibers) having the concentration agent particles enmeshed therein. Useful processes include meltblowing, spunbonding, and other air laying techniques; carding; wet laying; and combinations thereof (preferably, air laying, wet laying, and combinations thereof; more preferably, wet laying).

Fibers that are suitable for use in preparing the porous fibrous nonwoven matrix of the concentration device include pulpable fibers. Preferred pulpable fibers are those that are stable to radiation and/or to a variety of solvents. Fibers include combinations of polymeric fibers and inorganic fibers. Preferably, at least some of the fibers that are utilized exhibit a degree of hydrophilicity.

Suitable polymeric fibers include those made from natural (animal or vegetable) and/or synthetic polymers, including thermoplastic and solvent-dispersible polymers. Useful polymers include wool; silk; cellulosic polymers (for example, cellulose, cellulose derivatives); fluorinated polymers (for example, poly(vinyl fluoride), poly(vinylidene fluoride), copolymers of vinylidene fluoride such as poly(vinylidene fluoride-*co*-hexafluoropropylene), copolymers of chlorotrifluoroethylene such as poly(ethylene-*co*-chlorotrifluoroethylene)); chlorinated polymers; polyolefins (for example, poly(ethylene), poly(propylene), poly(1-butene), copolymers of ethylene and propylene, alpha olefin copolymers such as copolymers of ethylene or propylene with 1-butene, 1-hexene, 1-octene, and 1-decene, poly(ethylene-*co*-1-butene), poly(ethylene-*co*-1-butene-*co*-1-hexene)); poly(isoprenes); poly(butadienes); polyamides (for example, nylon 6; nylon 6,6; nylon 6,12; poly(iminoadipoyliminohexamethylene); poly(iminoadipoyliminodecamethylene); polycaprolactam); polyimides (for example, poly(pyromellitimide)); polyethers; poly(ether sulfones) (for example, poly(diphenylether sulfone), poly(diphenylsulfone-*co*-diphenylene oxide sulfone)); poly(sulfones); poly(vinyl acetates); copolymers of vinyl acetate (for example, poly(ethylene-*co*-vinyl acetate), copolymers in which at least some of the acetate groups have been hydrolyzed to provide various poly(vinyl alcohols) including poly(ethylene-*co*-vinyl alcohol)); poly(phosphazenes); poly(vinyl esters); poly(vinyl ethers); poly(vinyl alcohols); polyaramids (for example, para-aramids such as poly(paraphenylene terephthalamide) and fibers sold under the trade designation "KEVLAR" by DuPont Co., Wilmington, DE, pulps of which are commercially available in various grades based on the length of the fibers that make up the pulp such as, for example, "KEVLAR 1F306" and "KEVLAR 1F694", both of which include aramid fibers that are at least 4 mm in length); poly(carbonates); and combinations thereof. Preferred polymeric fibers include polyamides, polyolefins, polysulfones, and combinations thereof (more preferably, polyamides, polyolefins, and combinations thereof; most preferably, nylons, poly(ethylene), and combinations thereof).

Suitable inorganic fibers include those that comprise at least one inorganic material selected from glasses, ceramics, and combinations thereof. Useful inorganic fibers include fiberglasses (for example, E-glass, S-glass), ceramic fibers (for example, fibers made of metal oxides (such as alumina), silicon carbide, boron nitride, boron carbide), and combinations thereof. Useful ceramic fibers can be at least partially crystalline (exhibiting a discernible X-ray powder diffraction pattern or containing both crystalline and amorphous (glass) phases). Preferred inorganic fibers include fiberglasses and combinations thereof.

The fibers used to form the porous fibrous nonwoven matrix can be of a length and diameter that can provide a matrix having sufficient structural integrity and sufficient porosity for a particular application (for example, for a particular type of sample matrix). For example, lengths of at least about 0.5 mm, 1 mm, 2 mm, 3 mm, 4 mm, 6 mm, 8 mm, 10 mm, 15 mm, 20 mm, 25 mm, or even 30 mm (and combinations thereof), and diameters of at least about 10 $\mu$m (micrometer), 20 $\mu$m, 40 $\mu$m, or even 60 $\mu$m (and combinations thereof) can be useful. Preferred fiber lengths and diameters will vary, depending upon factors including the nature of the fiber and the type of application. For example, fibrillated poly(ethylene) can be useful in lengths of about 1 mm to about 3 mm, and non-fibrillated nylon can be useful in lengths of about 6 mm to about 12.5 mm, for a variety of sample matrices.

To facilitate entrapment of the concentration agent particles and/or to ensure a high surface area matrix, the fibers used to form the porous fibrous nonwoven matrix preferably comprise at least one fibrillated fiber (for example, in the form of a main fiber surrounded by many smaller attached fibrils). The main fiber generally can have a length in the range of about 0.5 mm to about 4 mm and a diameter of about 1 to about 20 micrometers. The fibrils typically can have a submicrometer diameter.

The porous fibrous nonwoven matrix can comprise two, three, four, or even more different types of fibers. For example, a nylon fiber can be added for strength and integrity, while fibrillated polyethylene can be added for entrapment of the particulates. If fibrillated and non-fibrillated fibers are used, generally the weight ratio of fibrillated fibers to non-fibrillated fibers can be at least about 1:2, 1:1, 2:1, 3:1, 5:1, or even 8:1. Regardless of the type(s) of fibers chosen, the amount of fiber in the resulting concentration device (in dry form) is preferably at least about 10, 12, 12.5, 14, 15, 18, 20, or even 22 percent by weight up to about 20, 25, 27, 30, 35, or even 40 percent by weight (based upon the total weight of all components of the concentration device).

Preferably, the porous fibrous nonwoven matrix further comprises at least one polymeric binder. Suitable polymeric binders include natural and synthetic polymeric materials that are relatively inert (exhibiting little or no chemical interaction with either the fibers or the concentration agent particles). Useful polymeric binders include polymeric resins (for example, in the form of powders and latexes), polymeric binder fibers, and combinations thereof. For at least some applications, preferred polymeric binders include polymeric binder fibers and combinations thereof. For other applications, polymeric resins and combinations thereof can be preferred polymeric binders.

Suitable polymeric resins include, but are not limited to, natural rubbers, neoprene, styrene-butadiene copolymers, acrylate resins, polyvinyl chloride, polyvinyl acetate, and combinations thereof. Preferred polymeric resins include acrylate resins and combinations thereof. Suitable polymeric binder fibers include adhesive-only type fibers (for example, Kodel™ 43UD fibers, available from Eastman Chemical Products, Kingsport, TN), bicomponent fibers (for example, side-by-side forms such as Chisso ES polyolefin thermally bonded bicomponent fibers, available from Chisso Corporation, Osaka, Japan; sheath-core forms such as Melty™ Fiber Type 4080 bicomponent fibers having a polyester core and a polyethylene sheath, available from Unitika Ltd., Osaka, Japan), and combinations thereof. Preferred polymeric binder fibers include bicomponent fibers and combinations thereof (more preferably, sheath-core bicomponent fibers and combinations thereof).

Regardless of the type of polymeric binder used, the amount of binder in the resulting concentration device (in dry form) generally can be from about 3 weight percent to about 7 weight percent (preferably, about 5 weight percent), based upon the total weight of all components of the concentration device. Such amounts of polymeric binder generally can provide the porous fibrous nonwoven matrix with sufficient integrity for use in many applications, while not significantly coating the particles. Surprisingly, the amount of polymeric binder in the concentration device can be less than about 5, 4, 3, 2, or even 1 percent by weight, relative to the weight of the fibers in the concentration device.

In preferred embodiments of the concentration device, the polymeric binder does not substantially adhere to the particles. In other words, when the concentration device is examined by scanning electron microscopy, less than about 5, 4, 3, 2, or even 1 percent of the total surface area of the particles is covered with polymeric binder.

The concentration device used in the process of the invention can be prepared by a process comprising (a) providing a plurality of the above-described fibers; (b) providing a plurality of the above-described concentration agent particles; and (c) forming at least a portion of the plurality of fibers into a porous fibrous nonwoven matrix having at least a portion of the plurality of particles enmeshed therein. As mentioned above, the forming can be carried out by essentially any process that is capable of providing a fibrous nonwoven matrix (that is, a web or medium, other than a woven or knitted fabric, comprising interlaid fibers) having the concentration agent particles enmeshed therein. Useful processes include meltblowing, spunbonding, and other air laying techniques; carding; wet laying; and combinations thereof (preferably, air laying, wet laying, and combinations thereof; more preferably, wet laying).

Preferably, the forming is carried out by using a wet laying or "wetlaid" process comprising (a) forming a dispersion comprising the plurality of fibers, the plurality of particles (which can be added and dispersed along with the other components prior to carrying out other process steps or, if desired, can be added and dispersed later in the process but generally prior to removal of dispersing liquid), and at least one polymeric binder in at least one dispersing liquid (preferably, water); (b) at least partially depositing the polymeric binder onto at least a portion of the fibers; and (c) removing the dispersing liquid from the dispersion. In such a process, the fibers can be dispersed in the dispersing liquid to form a slurry. If desired, the fibers can comprise additives or chemical groups or moieties to assist in their dispersion.

For example, polyolefin-based fibers can comprise maleic anhydride or succinic anhydride functionality, or, during the melt-processing of polyethylene fibers, a suitable surfactant can be added.

Deposition of the polymeric binder onto the fibers can be carried out either before or after the dispersing liquid removal or dewatering step, depending upon the nature of the polymeric binder. For example, when a polymeric latex is used as the polymeric binder, the polymeric latex can be precipitated onto the fibers before or after particle addition and prior to dewatering. After the dewatering, heat can be applied to finish the dewatering and to set the resulting deposited latex. When polymeric binder fibers are used as the polymeric binder, dewatering can generally be carried out first, followed by heating to finish the dewatering and to melt the polymeric binder fibers (and thereby deposit polymeric binder on the fibers).

One or more adjuvants or additives can be used in preparing the concentration device. Useful adjuvants include process aids (for example, precipitation agents such as sodium aluminate and aluminum sulfate, which can aid in precipitating the polymeric binder onto the fibers), materials that can enhance the overall performance of the resulting concentration device. When used, the amounts of such adjuvants can range from more than zero up to about 2 weight percent (preferably, up to about 0.5 weight percent; based upon the total weight of the components of the concentration device), although their amounts are preferably kept as low as possible so as to maximize the amount of concentration agent particles that can be included.

In a preferred wetlaid process, the fibers (for example, chopped fibers) can be blended in a container in the presence of the dispersing liquid (for example, water, a water-miscible organic solvent such as an alcohol, or a combination thereof). The amount of shear used to blend the resulting mixture has not been found to affect the ultimate properties of the resulting concentration device, although the amount of shear introduced during blending is preferably relatively high. Thereafter, the particles, the polymeric binder, and an excess of a precipitation agent (for example, a pH adjusting agent such as alum) can be added to the container.

When the preferred wetlaid process is carried out by using hand-sheet methods known in the art, the order of addition of the three ingredients to the fiber dispersion has not been found to significantly affect the ultimate performance of the concentration device. Addition of the polymeric binder after addition of the particles, however, can provide a concentration device exhibiting somewhat greater adhesion of the particles to the fibers. When the preferred wetlaid process is carried out by using a continuous method, the three ingredients preferably are added in the listed order. (The following description is based on a hand-sheet method, although those skilled in the art can readily recognize how to adapt such a method to provide for a continuous process.)

After the particles, the polymeric binder, and the precipitation agent are added to the fiber-liquid slurry, the resulting mixture can be poured into a mold, the bottom of which can be covered by a screen. The dispersing liquid (preferably, water) can be allowed to drain from the mixture (in the form of a wet sheet) through the screen. After sufficient liquid has drained from the sheet, the wet sheet generally can be removed from the mold and dried by pressing, heating, or a combination of the two. Generally pressures of about 300 to about 600 kPa and temperatures of about 100 to about 200°C (preferably, about 100 to about 150°C) can be used in these drying processes. When polymeric binder fibers are used as the polymeric binder in the preferred wetlaid process, no precipitation agent is needed, and the applied heat can be used to melt the polymeric binder fibers.

The resulting dry sheet can have an average thickness of at least about 0.2, 0.5, 0.8, 1, 2, 4, or even 5 mm up to about 5, 8, 10, 15, or even 20 mm. Up to about 100 percent of the dispersing liquid can be removed (preferably, up to about 90 percent by weight). Calendering can be used to provide additional pressing or fusing, if desired.

As mentioned above, the concentration agent particles can be microparticles. The microparticles can be entrapped in the porous fibrous nonwoven matrix through either chemical interactions (for example, chemical bonding) or physical interactions (for example, adsorption or mechanical entrapment), depending upon the nature of the fibers that are utilized. Preferred embodiments of the concentration device include those comprising at least one fibrillated fiber that can effect mechanical entrapment of the concentration agent particles. In one embodiment of the concentration device, the effective average diameter of the particles is at least about 175 times smaller than the uncalendered thickness of the resulting wetlaid sheet (preferably, at least about 250 times smaller than the uncalendered thickness of the sheet; more preferably, at least about 300 times smaller than the uncalendered thickness of the sheet).

Since the capacity and efficiency of the concentration device can vary according to the amount of concentration agent particles contained therein, relatively high particle loadings generally can be desirable. The amount of particles in the concentration device preferably can be at least about 20, 30, 40, 50, 60, 70, or even 80 weight percent (based upon the total weight of all components of the concentration device). The particles are entrapped in the porous fibrous nonwoven matrix and preferably distributed within it (more preferably, the particles are distributed essentially uniformly throughout the matrix).

The resulting concentration device can have controlled porosity (preferably, having a Gurley time of at least about 0.1 second (more preferably, at least about 2 to about 4 seconds; most preferably, at least about 4 seconds) for 100 mL of air). The basis weight of the concentration device (in the form of sheet material) can be in the range of about 250 to about 5000 g/m$^2$ (preferably, in the range of about 400 to about 1500 g/m$^2$; more preferably, about 500 to about

1200 g/m$^2$).

Generally the average pore size of the sheet material can be in the range of about 0.1 to about 10 micrometers, as measured by scanning electron microscopy (SEM). Void volumes in the range of about 20 to about 80 volume percent can be useful (preferably, about 40 to about 60 volume percent). The porosity of the sheet materials can be modified (increased) by including fibers of larger diameter or stiffness in the fiber mixture.

The sheet material can be flexible (for example, able to be rolled around a 0.75 inch (about 2 cm) diameter core). This flexibility can enable the sheet material to be pleated or rolled. The sheet material can have a relatively low back pressure (meaning that a relatively high volume of liquid can be relatively quickly passed through it without generating relatively high back pressure). (As used herein, "relatively low back pressure" refers to a differential back pressure of less than about 3 pounds per square inch (20.7 kPa), 2.5 (17.2), 2 (13.8), 1.5 (10.3), or even 1 pound per square inch (6.9 kPa) at a 3 mL/cm$^2$ flowrate, wherein the flowrate is based on the frontal surface area of the sheet material.)

The uncalendered sheet material can be cut to a desired size and used to carry out the concentration process of the invention. If desired (for example, when a significant pressure drop across the sheet is not a concern), the sheet material can be calendered to increase its tensile strength prior to use. When the sheet material is to be pleated, drying and calendering preferably can be avoided.

A single layer of sheet material can be effective in carrying out the concentration process of the invention. Multiple layers can be used, if desired, to provide greater concentration capacity.

A significant advantage of the porous fibrous nonwoven matrix of the concentration device is that very small concentration agent particle sizes (10 μm or smaller) and/or concentration agent particles with a relatively broad size distribution can be employed. This allows for excellent one-pass kinetics, due to increased surface area/mass ratios and, for porous particles, minimized internal diffusion distances. Because of the relatively low pressure drops, a minimal driving force (such as gravity or a vacuum) can be used to pull a sample through the concentration device, even when small concentration agent particle sizes are employed.

If desired, the concentration device can further comprise one or more other components such as, for example, one or more pre-filters (for example, to remove relatively large food particles from a sample prior to passage of the sample through the porous matrix), a support or base for the porous matrix (for example, in the form of a frit or grid), a manifold for applying a pressure differential across the device (for example, to aid in passing a sample through the porous matrix), and/or an external housing (for example, a disposable cartridge to contain and/or protect the porous matrix).

## Sample

The process of the invention can be applied to a variety of different types of samples, including, but not limited to, medical, environmental, food, feed, clinical, and laboratory samples, and combinations thereof. Medical or veterinary samples can include, for example, cells, tissues, or fluids from a biological source (for example, a human or an animal) that are to be assayed for clinical diagnosis. Environmental samples can be, for example, from a medical or veterinary facility, an industrial facility, soil, a water source, a food preparation area (food contact and non-contact areas), a laboratory, or an area that has been potentially subjected to bioterrorism. Food processing, handling, and preparation area samples are preferred, as these are often of particular concern in regard to food supply contamination by bacterial pathogens.

Samples obtained in the form of a liquid or in the form of a dispersion or suspension of solid in liquid can be used directly, or can be concentrated (for example, by centrifugation) or diluted (for example, by the addition of a buffer (pH-controlled) solution). Samples in the form of a solid or a semi-solid can be used directly or can be extracted, if desired, by a method such as, for example, washing or rinsing with, or suspending or dispersing in, a fluid medium (for example, a buffer solution). Samples can be taken from surfaces (for example, by swabbing or rinsing). Preferably, the sample is a fluid (for example, a liquid, a gas, or a dispersion or suspension of solid or liquid in liquid or gas).

Examples of samples that can be used in carrying out the process of the invention include foods (for example, fresh produce or ready-to-eat lunch or "deli" meats), beverages (for example, juices or carbonated beverages), water (including potable water), and biological fluids (for example, whole blood or a component thereof such as plasma, a platelet-enriched blood fraction, a platelet concentrate, or packed red blood cells; cell preparations (for example, dispersed tissue, bone marrow aspirates, or vertebral body bone marrow); cell suspensions; urine, saliva, and other body fluids; bone marrow; lung fluid; cerebral fluid; wound exudate; wound biopsy samples; ocular fluid; spinal fluid), as well as lysed preparations, such as cell lysates, which can be formed using known procedures such as the use of lysing buffers. Preferred samples include foods, beverages, water, biological fluids, and combinations thereof (with foods, beverages, water, and combinations thereof being more preferred, and with water being most preferred).

Sample volume can vary, depending upon the particular application. For example, when the process of the invention is used for a diagnostic or research application, the volume of the sample can typically be in the microliter range (for example, 10 microliters or greater). When the process is used for a food pathogen testing assay or for potable water safety testing, the volume of the sample can typically be in the milliliter to liter range (for example, 100 milliliters to 3 liters). In an industrial application, such as bioprocessing or pharmaceutical formulation, the volume can be tens of thousands of liters.

The process of the invention can isolate microorganisms from a sample in a concentrated state and can also allow the isolation of microorganisms from sample matrix components that can inhibit detection procedures that are to be used. In all of these cases, the process of the invention can be used in addition to, or in replacement of, other methods of cellular analyte or microorganism concentration. Thus, optionally, cultures can be grown from samples either before or after carrying out the process of the invention, if additional concentration is desired. Such cultural enrichment can be general or primary (so as to enrich the concentrations of most or essentially all microorganisms) or can be specific or selective (so as to enrich the concentration(s) of one or more selected microorganisms only).

**Contacting**

The process of the invention can be carried out by any of various known or hereafter-developed methods of providing contact between two materials. For example, the concentration device can be added to the sample, or the sample can be added to the concentration device. The concentration device can be immersed in a sample, a sample can be poured onto the concentration device, a sample can be poured into a tube or well containing the concentration device, or, preferably, a sample can be passed over or through (preferably, through) the concentration device (or vice versa). Preferably, the contacting is carried out in a manner such that the sample passes through at least one pore of the porous fibrous nonwoven matrix (preferably, through at least one through pore).

The concentration device and the sample can be combined (using any order of addition) in any of a variety of containers or holders (optionally, a capped, closed, or sealed container; preferably, a column, a syringe barrel, or another holder designed to contain the device with essentially no sample leakage). Suitable containers for use in carrying out the process of the invention will be determined by the particular sample and can vary widely in size and nature. For example, the container can be small, such as a 10 microliter container (for example, a test tube or syringe) or larger, such as a 100 milliliter to 3 liter container (for example, an Erlenmeyer flask or an annular cylindrical container).

The container, the concentration device, and any other apparatus or additives that contact the sample directly can be sterilized (for example, by controlled heat, ethylene oxide gas, or radiation) prior to use, in order to reduce or prevent any contamination of the sample that might cause detection errors. The amount of concentration agent in the concentration device that is sufficient to capture or concentrate the microorganisms of a particular sample for successful detection will vary (depending upon, for example, the nature and form of the concentration agent and device and the volume of the sample) and can be readily determined by one skilled in the art.

Contacting can be carried out for a desired period (for example, for sample volumes of several liters or for processes involving multiple passes through the concentration device, up to about 60 minutes of contacting can be useful; preferably, about 15 seconds to about 10 minutes or longer; more preferably, about 15 seconds to about 5 minutes; most preferably, about 15 seconds to about 2 minutes). Contact can be enhanced by mixing (for example, by stirring, by shaking, or by application of a pressure differential across the device to facilitate passage of a sample through its porous matrix) and/or by incubation (for example, at ambient temperature), which are optional but can be preferred, in order to increase microorganism contact with the concentration device.

Preferably, contacting can be effected by passing a sample at least once (preferably, only once) through the concentration device (for example, by pumping). Essentially any type of pump (for example, a peristaltic pump) or other equipment for establishing a pressure differential across the device (for example, a syringe or plunger) can be utilized. Useful flow rates will vary, depending upon such factors as the nature of the sample matrix and the particular application.

For example, sample flow rates through the device of up to about 100 milliliters per minute or more can be effective. Preferably, for samples such as beverages and water, flow rates of about 10-20 milliliters per minute can be utilized. For pre-filtered or otherwise clarified food samples, flow rates of about 6 milliliters per minute (1.5 milliliters per 15 seconds) can be useful. Longer contact times and slower flow rates can be useful for more complex sample matrices such as ground beef or turkey.

A preferred contacting method includes such passing of a sample through the concentration device (for example, by pumping). If desired, one or more additives (for example, lysis reagents, bioluminescence assay reagents, nucleic acid capture reagents (for example, magnetic beads), microbial growth media, buffers (for example, to moisten a solid sample), microbial staining reagents, washing buffers (for example, to wash away unbound material), elution agents (for example, serum albumin), surfactants (for example, Triton™ X-100 nonionic surfactant available from Union Carbide Chemicals and Plastics, Houston, TX), mechanical abrasion/elution agents (for example, glass beads), adsorption buffers (for example, the same buffer used for preparing the above-mentioned adsorption buffer-modified concentration agent or a different buffer)) can be included in the combination of concentration device and sample during contacting.

The process of the invention can optionally further comprise separating the resulting target cellular analyte-bound concentration device and the sample. Separation can be carried out by numerous methods that are well-known in the art (for example, by pumping, decanting, or siphoning a fluid sample, so as to leave the target cellular analyte-bound concentration device in the container or holder utilized in carrying out the process). It can also be possible to isolate or separate captured target cellular analytes (target microorganisms or one or more components thereof) from the concentration device after sample contacting (for example, by passing an elution agent or a lysis agent over or through the concentration device).

The process of the invention can be carried out manually (for example, in a batchwise manner) or can be automated (for example, to enable continuous or semi-continuous processing).

## Detection

A variety of microorganisms can be concentrated and detected by using the process of the invention, including, for example, bacteria, fungi, yeasts, protozoans, viruses (including both non-enveloped and enveloped viruses), bacterial endospores (for example, *Bacillus* (including *Bacillus anthracis, Bacillus cereus, and Bacillus subtilis*) and *Clostridium* (including *Clostridium botulinum, Clostridium difficile, and Clostridium perfringens*)), and combinations thereof (preferably, bacteria, yeasts, viruses, bacterial endospores, fungi, and combinations thereof; more preferably, bacteria, yeasts, bacterial endospores, fungi, and combinations thereof; even more preferably, bacteria, yeasts, fungi, and combinations thereof; still more preferably, gram-negative bacteria, gram-positive bacteria, yeasts, fungi, and combinations thereof; most preferably, gram-negative bacteria, gram-positive bacteria, yeasts, and combinations thereof). The process has utility in the detection of pathogens, which can be important for food safety or for medical, environmental, or anti-terrorism reasons. The process can be particularly useful in the detection of pathogenic bacteria (for example, both gram negative and gram positive bacteria), as well as various yeasts and molds (and combinations of any of these).

Genera of target microorganisms to be detected include, but are not limited to, *Listeria, Escherichia, Salmonella, Campylobacter, Clostridium, Helicobacter, Mycobacterium, Staphylococcus, Shigella, Enterococcus, Bacillus, Neisseria, Shigella, Streptococcus, Vibrio, Yersinia, Bordetella, Borrelia, Pseudomonas, Saccharomyces, Candida,* and combinations thereof. Samples can contain a plurality of microorganism strains, and any one strain can be detected independently of any other strain. Specific microorganism strains that can be targets for detection include *Escherichia coli, Yersinia enterocolitica, Yersinia pseudotuberculosis, Vibrio cholerae, Vibrio parahaemolyticus, Vibrio vulnificus, Listeria monocytogenes* (for which *Listeria innocua* is a surrogate), *Staphylococcus aureus, Salmonella enterica, Saccharomyces cerevisiae, Candida albicans, Staphylococcal enterotoxin* ssp, *Bacillus cereus, Bacillus anthracis, Bacillus atrophaeus, Bacillus subtilis, Clostridium perfringens, Clostridium botulinum, Clostridium difficile, Enterobacter sakazakii,* human-infecting non-enveloped enteric viruses for which *Escherichia coli* bacteriophage is a surrogate, *Pseudomonas aeruginosa,* and combinations thereof (preferably, *Staphylococcus aureus, Listeria monocytogenes* (for which *Listeria innocua* is a surrogate), *Salmonella enterica, Saccharomyces cerevisiae, Bacillus subtilis, Pseudomonas aeruginosa, Escherichia coli,* human-infecting non-enveloped enteric viruses for which *Escherichia coli* bacteriophage is a surrogate, and combinations thereof; more preferably, *Staphylococcus aureus, Listeria monocytogenes* (for which *Listeria innocua* is a surrogate), *Saccharomyces cerevisiae, Pseudomonas aeruginosa,* and combinations thereof).

Microorganisms that have been captured or bound (for example, by adsorption or by sieving) by the concentration device can be detected by essentially any desired method that is currently known or hereafter developed. Such methods include, for example, culture-based methods (which can be preferred when time permits), microscopy (for example, using a transmitted light microscope or an epifluorescence microscope, which can be used for visualizing microorganisms tagged with fluorescent dyes) and other imaging methods, immunological detection methods, and genetic detection methods. The detection process following microorganism capture optionally can include washing to remove sample matrix components, slicing or otherwise breaking up the porous fibrous nonwoven matrix of the concentration device, staining, boiling or using elution buffers or lysis agents to release cellular analyte from the concentration device.

Immunological detection is detection of an antigenic material derived from a target organism, which is commonly a biological molecule (for example, a protein or proteoglycan) acting as a marker on the surface of bacteria or viral particles. Detection of the antigenic material typically can be by an antibody, a polypeptide selected from a process such as phage display, or an aptamer from a screening process.

Immunological detection methods are well-known and include, for example, immunoprecipitation and enzyme-linked immunosorbent assay (ELISA). Antibody binding can be detected in a variety of ways (for example, by labeling either a primary or a secondary antibody with a fluorescent dye, with a quantum dot, or with an enzyme that can produce chemiluminescence or a colored substrate, and using either a plate reader or a lateral flow device).

Detection can also be carried out by genetic assay (for example, by nucleic acid hybridization or primer directed amplification), which is often a preferred method. The captured or bound microorganisms can be lysed to render their genetic material available for assay. Lysis methods are well-known and include, for example, treatments such as sonication, osmotic shock, high temperature treatment (for example, from about 50°C to about 100°C), and incubation with an enzyme such as lysozyme, glucolase, zymolose, lyticase, proteinase K, proteinase E, and viral enolysins.

Many commonly-used genetic detection assays detect the nucleic acids of a specific microorganism, including the DNA and/or RNA. The stringency of conditions used in a genetic detection method correlates with the level of variation in nucleic acid sequence that is detected. Highly stringent conditions of salt concentration and temperature can limit the detection to the exact nucleic acid sequence of the target. Thus microorganism strains with small variations in a target nucleic acid sequence can be distinguished using a highly stringent genetic assay. Genetic detection can be based on nucleic acid hybridization where a single-stranded nucleic acid probe is hybridized to the denatured nucleic acids of the microorganism such that a double-stranded nucleic acid is produced, including the probe strand. One skilled in the art will be familiar with probe labels, such as radioactive, fluorescent, and chemiluminescent labels, for detecting the hybrid following gel electrophoresis, capillary electrophoresis, or other separation method.

Particularly useful genetic detection methods are based on primer directed nucleic acid amplification. Primer directed nucleic acid amplification methods include, for example, thermal cycling methods (for example, polymerase chain reaction (PCR), reverse transcriptase polymerase chain reaction (RT-PCR), and ligase chain reaction (LCR)), as well as isothermal methods and strand displacement amplification (SDA) (and combinations thereof; preferably, PCR or RT-PCR). Methods for detection of the amplified product are not limited and include, for example, gel electrophoresis separation and ethidium bromide staining, as well as detection of an incorporated fluorescent label or radio label in the product. Methods that do not require a separation step prior to detection of the amplified product can also be used (for example, real-time PCR or homogeneous detection).

Bioluminescence detection methods are well-known and include, for example, adensosine triphosphate (ATP) detection methods including those described in U.S. Patent No. 7,422,868 (Fan et al.). Other luminescence-based detection methods can also be utilized.

Since the process of the invention is non-strain specific, it provides a general capture system that allows for multiple microorganism strains to be targeted for assay in the same sample. For example, in assaying for contamination of food samples, it can be desired to test for *Listeria monocytogenes, Escherichia coli*, and *Salmonella* all in the same sample. A single capture step can then be followed by, for example, PCR or RT-PCR assays using specific primers to amplify different nucleic acid sequences from each of these microorganism strains. Thus, the need for separate sample handling and preparation procedures for each strain can be avoided.

### Diagnostic Kit

A diagnostic kit for use in carrying out the concentration process of the invention comprises (a) at least one above-described concentration device; and (b) at least one testing container or testing reagent (preferably, a sterile testing container or testing reagent) for use in carrying out the concentration process of the invention. Preferably, the diagnostic kit further comprises instructions for carrying out the process.

Useful testing containers or holders include those described above and can be used, for example, for contacting, for incubation, for collection of eluate, or for other desired process steps. Useful testing reagents include microorganism culture or growth media, lysis agents, elution agents, buffers, luminescence detection assay components (for example, luminometer, lysis reagents, luciferase enzyme, enzyme substrate, reaction buffers), genetic detection assay components, and combinations thereof. A preferred lysis agent is a lytic enzyme or chemical supplied in a buffer, and preferred genetic detection assay components include one or more primers specific for a target microorganism. The kit can optionally further comprise sterile forceps.

### EXAMPLES

Objects and advantages of this invention are further illustrated by the following examples, but the particular materials and amounts thereof recited in these examples, as well as other conditions and details, should not be construed to unduly limit this invention. All parts, percentages, ratios, and so forth, in the following examples are by weight, unless noted otherwise. Solvents and other reagents were obtained from Sigma-Aldrich Chemical Company, Milwaukee, WI, unless specified differently. All microorganism cultures were purchased from The American Type Culture Collection (ATCC; Manassas, VA). Experimental results are an average of 2 tests, unless otherwise stated. Overnight cultures were prepared by streaking selected microorganisms on Tryptic Soy Agar plates and then incubating the plates at 37°C overnight. All microorganism counts were performed according to standard microbiological counting procedures for colony forming units, and counts are approximate numbers.

### Concentration Agents

Crystalline magnesium silicate concentration agent (hereinafter, Talc) was purchased from Mallinckrodt Baker, Inc. (Phillipsburg, NJ).

Amorphous, spheroidized magnesium silicate concentration agent (hereinafter, AS-Talc) was obtained as 3M™ Cosmetic Microspheres CM-111 (shaped as solid spheres; particle density of 2.3 g/cubic centimeter; surface area of

3.3 m$^2$/g; particle size: 90 percent less than about 11 microns, 50 percent less than about 5 microns, 10 percent less than about 2 microns; available from 3M Company, St. Paul, MN).

**Zeta Potential Measurements**

Zeta potentials of aqueous dispersions of the Talc and AS-Talc concentration agents (5.75 weight percent Talc and 5.8 weight percent AS-Talc, respectively, in 18 mega ohms deionized water obtained by using a Milli-Q™ Elix 10™ Synthesis A10 deionization system from Millipore Corporation, Bedford, MA) were measured as a function of added hydrochloric acid (pH) using a Colloidal Dynamics Acoustosizer II™ multi-frequency electroacoustic spectral analyzer (Colloidal Dynamics, Warwick, RI) equipped with a TM200 automatic titration module, pH electrode, and in-line conductivity cell. Measurements were made using polar calibration and polar sample settings with the following general parameters:

| | |
|---|---|
| **Starting Volume:** | 170 mL of dispersion |
| **Titration Volume:** | 5 to 10 mL at finish; 20 steps for each titration |
| **Titrant:** | 1.0 N hydrochloric acid in water (J.T. Baker, Phillipsburg, NJ) |
| **Stir Rate:** | 300 revolutions per minute (rpm) |
| **Pump Rate:** | 400 mL per minute |
| **Mixing Delay:** | 120 seconds with stirring after acid addition before measurement |

At a pH of about 7, the AS-Talc exhibited a Smoluchowski zeta potential of about -12 mV, and the Talc exhibited a Smoluchowski zeta potential of about -8 mV.

**Surface Composition Analysis**

The surface compositions of samples of the Talc and AS-Talc concentration agents were analyzed by X-ray photoelectron spectroscopy (XPS; also known as ESCA). Samples of the powders were pressed onto double-sided, pressure sensitive adhesive tapes on aluminum foil. Excess powder was removed from each sample surface by blowing with compressed nitrogen gas.
Spectral data was acquired using a Kratos AXIS Ultra™ DLD spectrometer (Kratos Analytical, Manchester, England) having a monochromatic Al-K$_\alpha$ X-ray excitation source (1487 eV) and a hemispherical electron energy analyzer operated in a constant pass energy mode. The emitted photoelectrons were detected at a take-off angle of 90 degrees measured with respect to the sample surface with a solid angle of acceptance of $\pm$ 10 degrees. A low-energy electron flood gun was used to minimize surface charging. Measurements were made using a 140 Watt power to anode and 2 x 10$^{-8}$ Torr chamber pressure.
An area of the surface of each concentration agent sample measuring about 300 micrometers by about 700 micrometers was analyzed for each data point. Three areas on each sample were analyzed and averaged to obtain the reported average atomic percent values. Data processing was carried out using standard Vision2™ software (Kratos
Analytical, Manchester, England). Results (elements present at a detectable level by XPS on the surface of the concentration agents) are shown in Table A below:

**TABLE A.**

| Concentration Agent | Magnesium (Average Atomic Percent) | Silicon (Average Atomic Percent) | Ratio of Magnesium to Silicon | Carbon (Average Atomic Percent) | Oxygen (Average Atomic Percent) |
|---|---|---|---|---|---|
| Talc | 17 | 26 | 0.65 | 6.9 | 50 |
| AS-Talc | 6.5 | 32 | 0.20 | 14 | 47 |

**Materials:**

■ All bacterial and yeast stock cultures (Saccharomyces cerevisiae (ATCC 201390), Listeria monocytogenes (ATCC 51414), Escherichia coli (ATCC 51813), Pseudomonas aeruginosa (ATCC 9027), Staphylococcus aureus (ATCC 6538), and Listeria innocua (ATCC 33090)) were purchased from The American Type Culture Collection, Manassas, VA, unless stated otherwise. Microorganisms for testing were isolated from a streak culture prepared by streaking a stock culture on a Tryptic Soy Agar plate and incubating overnight at 37°C according to standard microbiology practices.

- The following fibers were obtained from Minifibers, Inc., Johnson City, TN

  - Fiber 1 - 1 denier fibrillated polyethylene fibers (FYBREL™ 620)

  - Fiber 2 - fibrillated polyethylene fibers (FYBREL™ 400)

  - Fiber 3 - 6 denier 3.125 mm (0.125 inch) length chopped nylon fibers

  - Fiber 4 - 6 denier 6.25 mm (0.25 inch) length chopped nylon fibers

  - Fiber 5 - 6 denier 12.5 mm (0.5 inch) length chopped nylon fibers

  - Fiber 7 - 1 denier bicomponent ethylene vinyl acetate (sheath)/polypropylene (core) fibers

- Fiber 6 - long glass fibers (Micro-Strand 106-475 Glass fiberglass from Schuller, Inc., Denver, CO)
- Latex binder - 50 weight percent (wt %) solids vinyl acetate emulsion purchased as Airflex 600BP from Air Products Polymers, Allentown, PA
- Flocculant - MP 9307 Flocculant (believed to be an aqueous solution of a copolymer of dimethylamine and epichlorohydrin), Midsouth Chemical Co., Inc., Riggold, LA
- AS-Talc - amorphous magnesium silicate spheroids (the above-described 3M™ Cosmetic Microspheres CM-111 from 3M Company, St. Paul, MN)
- BHI Broth - DIFCO™ Bovine Heart Infusion Broth general-purpose growth medium from Becton Dickinson, Sparks, MD, prepared at 3.7 weight percent (wt %) concentration according to the manufacturer's instructions
- Buffer solution - Butterfield's Buffer, pH 7.2 ± 0.2; monobasic potassium phosphate buffer solution; VWR Catalog Number 83008-093; VWR, West Chester, PA
- Tryptic Soy Agar plate - Difco™ Tryptic Soy Agar obtained from Becton Dickinson, Sparks, MD, prepared at 3 weight percent (wt %) according to the manufacturer's instructions using Difco™ Tryptic Soy Broth, Becton Dickinson, Sparks, MD
- MOX plate - Oxford Medium, Modified for Listeria, agar-based growth medium obtained from Hardy Diagnostics, Santa Maria, CA
- YPD agar plate - agar plate prepared according to manufacturer's instructions with 5 wt % Difco™ Yeast Extract Peptone Dextrose powder and 1.5 wt % Difco™ agar powder, both powders from Becton Dickinson, Sparks, MD
- E. coli plate - 3M™ Petrifilm™ E. coli/Coliform Count Plate (flat film culture devices comprising at least one fermentable nutrient); 3M Company, St. Paul, MN
- AC plate - 3M™ Petrifilm™ Aerobic Count Plate (a flat film culture device comprising dry, rehydratable culture medium); 3M Company, St. Paul, MN
- Y/M plates - 3M™ Petrifilm™ Yeast and Mold Count Plates (a flat film culture device comprising dry, rehydratable culture medium); 3M Company, St. Paul, MN
- PIA plates - Pseudomonas Isolation Agar from Teknova; purchased from VWR, West Chester, PA
- C-agar plates - BBL™ CHROMagar™ Staph aureus plates (agar-based growth medium; made by Becton Dickinson, purchased from VWR, West Chester, PA)
- Syringes - BD Luer-Lok™ Tip syringe puchased from VWR, West Chester, PA
- Elisa Assay - 3M™ TECRA™ Listeria Visual Immunoassay kit; 3M Company, St. Paul, MN
- Stomacher and stomacher bags - Stomacher™ 400 Circulator laboratory blender and Stomacher™ polyethylene filter bags, Seward Corp., Norfolk, UK; purchased from VWR, West Chester, PA

**Terms**

- Porous fibrous nonwoven matrix - may also be referred to as a dry felt, a pad, a matrix, a disk, or a filter in the following examples and comparative examples
- Solids Retention - The term "Solids Retention" refers to the weight percentage of total solids retained in a porous fibrous nonwoven matrix during a pad-making process. It was calculated by dividing the total weight of a finished, dry, porous nonwoven fiber matrix by the total weight of all of the solid materials used to make the matrix, except the flocculant (for example, the total weight of latex binder solids, fibers, and AS-Talc). ▪ CFUs - colony-forming units
▪ Colony count - Microorganism colonies were counted manually according to standard microbiology procedures, unless otherwise stated. All colony counts were approximate.
▪ Filtrate count - the count of microorganism colonies in a filtrate
▪ Pre-filtration count - the count of microorganism colonies in a pre-filtration sample
▪ MCE - Microorganism Capture Efficiency (or Binding Efficiency) of a porous fibrous nonwoven matrix is an assessment of how well the matrix captures microorganisms. The MCE, in percent (%), was determined by the following formula:

$$MCE = 100 - [(\text{Filtrate count}/\text{Pre-filtration count}) \times 100]$$

## Examples 1 - 17 and Comparative Examples C1 - C4: Preparation of Concentration Devices 1-17 and C1-C4

Fiber premixes were prepared having the compositions of fiber and water shown in Table 1. Each fiber premix was prepared by first blending a fibrillated fiber (Fiber 1 or Fiber 2) with the amount of cold tap water specified in a 4 L blender (Waring Commercial Heavy Duty Blender, Model 37BL84) at medium speed for 90 seconds. The fibers were examined to ensure that they were uniformly dispersed without nits or clumps and were blended further if necessary to break up any clumps. The other specified fibers were then added with further blending, and the amount of premix specified was then added to a stainless steel beaker and mixed with an impeller mixer (Fisher Scientific Stedfast Stirrer model SL2400, available from VWR, West Chester, PA) at a speed setting of 4 for five minutes. When used, latex binder was dispersed in about 25 mL of tap water in a 50 mL beaker and added to the premix. The 50 mL beaker was rinsed with another 25 ml of tap water, which was also added to the premix and the resulting mixture blended for about 2 minutes. When used, flocculant was likewise dispersed in about 25 mL of tap water in a beaker and added to the mixture while blending, followed by the addition of another 25 mL of rinse water from the beaker. The latex binder crashed out of solution onto the fibers, and the liquid phase of the premix changed from cloudy to substantially clear. Then, AS-Talc particles were added to the mixture and vortex mixed for 1 minute.

A felt was prepared using a TAPPI™ pad maker apparatus (Williams Apparatus, Watertown, NY). The apparatus had an enclosed box measuring about 20 centimeters (8 inches) square and 20 centimeters (8 inches) deep, with a fine mesh screen near the bottom and a drain valve below the screen. The box was filled with tap water to a height of about 1 cm above the screen. The particle-containing mixture was poured into the box, and the valve was opened immediately, creating a vacuum that pulled the water out of the box. The resulting wetlaid felt was approximately 3 mm thick.

The wetlaid felt was transferred from the apparatus onto a sheet of blotter paper (20 centimeters by 20 centimeters (8 inch by 8 inch) 96-pound white paper, Anchor Paper, St. Paul, MN). The felt was sandwiched between 2 - 4 layers of blotter paper, depending upon the wetness of the felt, and pressed between 2 reinforced screens in an air-powered press set at 413 kPa (60 psi) (calculated to be about 82.7 kPa (12 psi) pressure exerted on the felt) for 1-2 minutes until no further water was observed being expelled. The pressed felt was then transferred onto a fresh sheet of blotter paper and placed in an oven (Blue M, Blue Island, IL; Stabil-Therm™ model OV-560A2) set at 150°C for about 40 minutes to remove residual water and cure the latex binder and/or melt polymeric binder fibers.

For Examples 3-5 and C2, the wet felt was left in the pad maker, and a heavy (approximately 5 kg (10 pound)) stainless steel roller was rolled over the felt to dewater it. The felt was then blotted essentially according to the procedure described above.

Example 16 was prepared by essentially the above-described procedure, except that the AS-Talc particles were added to the fiber premix before addition of the latex binder.

The resulting porous fibrous nonwoven matrices were sealed in plastic bags and irradiated with gamma radiation at 0.5 kGy/hour for 8 hours for a total dose of 4kGy, or were autoclaved at 121°C for 15 minutes, to sterilize the matrices. Square sample matrices (measuring 20 centimeters by 20 centimeters (8 inches by 8 inches)) were cut from the matrices of selected examples, were weighed, and solids retention values were determined for these sample matrices, as shown in Table 2.

**TABLE 1.**

| Ex. No. | Water (L) | Fiber 1 (g) | Fiber 2 (g) | Fiber 3 (g) | Fiber 4 (g) | Fiber 5 (g) | Fiber 6 (g) | Fiber 7 (g) | Premix (mL) | Latex Binder (g) | Flocculant (g) | AS-Talc (g) |
|---|---|---|---|---|---|---|---|---|---|---|---|---|
| 1 | 3 | 12 | -- | 1.5 | 3 | -- | 3 | -- | 500 | 0.60 | 1.39 | 7.0 |
| 2 | 3 | 12 | -- | 1.5 | 3 | -- | 3 | -- | 500 | 0.78 | 1.79 | 14.4 |
| 3 | 4 | 16 | -- | -- | 6 | -- | 5 | -- | 500 | 1.0 | 2.0 | 7.0 |
| 4 | 4 | 16 | -- | -- | 6 | -- | 5 | -- | 500 | 1.24 | 2.14 | 10.0 |
| 5 | 4 | 16 | -- | -- | 6 | -- | 5 | -- | 500 | 1.05 | 2.11 | 14.0 |
| 6 | 4 | 16 | -- | -- | 6 | -- | 5 | -- | 1000 | 1.05 | 2.0 | 15.0 |
| 7 | 4 | 16 | -- | -- | 6 | -- | 5 | -- | 2000 | 2.00 | 4.00 | 10.0 |
| 8 | 1.25 | 7.5 | -- | -- | -- | 1.5 | 0.88 | 1.15 | All* | -- | -- | 5 |
| 9 | 4 | 16 | -- | -- | 6 | -- | 5 | -- | 1000 | 1.1 | 2.08 | 10 |
| 10 | 4 | 16 | -- | -- | 6 | -- | 5 | -- | 2000 | 2.1 | 4.1 | 20 |
| 11 | 4 | -- | 16 | -- | 7 | -- | 5.09 | -- | 3000 | 3.11 | 2.22 | 10.28 |
| 12 | 3 | -- | 22.5 | -- | -- | 5.25 | 3.75 | 4.5 | 1000 | 1 | 2 | 5 |
| 13 | 3 | -- | 22.5 | -- | -- | 5.25 | 3.75 | 4.5 | 1000 | 1 | 2 | 10 |
| 14 | 4 | -- | 16 | -- | 8.43 | -- | 4.26 | 6.12 | 500 | -- | -- | 4.9 |
| 15 | 1 | -- | 3.75 | -- | -- | 0.825 (g) | 0.6 | 0.75 | All* | -- | -- | 5 |
| 16 | 1 | 7.5 | | | | 1.5 | 0.9 | 1.1 | All* | 2.0 | 2.0 | 9.01 |
| 17 | 1 | 6 | -- | 2 | -- | 1.5 | 1 | -- | All* | 1.0 | 2.0 | 10 |
| C1 | 3 | 12 | -- | 1.5 | 3 | -- | 3 | -- | 500 | 0.66 | 1.30 | -- |
| C2 | 4 | 16 | -- | -- | 6 | -- | 5 | -- | 500 | 1.05 | 2.0 | -- |
| C3 | 4 | 16 | -- | -- | 6 | -- | 5 | -- | 1000 | 1.0 | 2.0 | -- |
| C4 | 3 | 22.5 | -- | -- | -- | 5.25 | 3.75 | 4.5 | 1000 | -- | -- | -- |

*All indicates that the entire batch of fiber premix was used to make the sample.

EP 2 649 193 B1

**TABLE 2.**

| Example No. | 1 | 3 | 4 | 5 | C2 |
|---|---|---|---|---|---|
| Solids Retention (%) | 80 | 80.7 | 80.9 | 82.1 | 89.9 |
| Dry Weight (g) | 14.04 | 8.47 | 10.92 | 14.37 | 3.53 |

**Examples 18-20 and Comparative Example C5: Testing of Concentration Devices 3-5 and C2**

A *Listeria innocua* colony, isolated from a streak culture, was inoculated into 5 mL BHI Broth and incubated at 30°C overnight (18-20 hours). The overnight culture of $10^8$ CFUs/mL (colony forming units/mL) was diluted in buffer solution and inoculated into 100 mL of BHI Broth to obtain a bacterial suspension having $10^4$ CFUs/mL ($10^6$ CFUs total).

Circular disks (48 mm diameter) were die-punched from the matrices of Examples 3, 5, and C2 and autoclaved at 121°C for 15 minutes to sterilize. A disk was placed on the membrane support of a positive pressure manual filtration device, and the support was attached to the filter body of the device. The device is described in International Publication No. WO2008/150779 (Figures 1A and 1B). The bacterial suspension was poured into the device and plunged to yield a filtrate. This procedure was carried out for each disk.

Two 100-microliter volumes of the filtrate and a pre-filtration control were diluted 1:10, 1:100, and 1:1000 with buffer solution, plated on MOX plates, and incubated at 37°C for 18-20 hours. Colonies were counted manually, and Microorganism Capture Efficiency (MCE) was calculated. Results are shown in Table 3.

**TABLE 3.**

| Example No. | 18 | 19 | 20 | C5 |
|---|---|---|---|---|
| Concentration Device | 3 | 4 | 5 | C2 |
| MCE (%) | 59 | 74 | 82 | 55 |

**Examples 21-24: Testing of Concentration Device 3**

Bacterial suspensions of *Listeria innocua,* having the concentrations shown in Table 4, were prepared from overnight cultures essentially according to the procedure of Example 18. The suspensions were filtered through sterilized 48 mm diameter disks of the matrix prepared in Example 3 using the positive pressure manual filtration device. Samples of the resulting filtrate and the suspension before filtering were diluted, plated, and incubated essentially according to the procedure of Example 18. Colonies were counted manually, and the Microorganism Capture Efficiency was calculated. Results are shown in Table 4.

**TABLE 4.**

| Example No. | 21 | 22 | 23 | 24 |
|---|---|---|---|---|
| Total CFUs in 100 mL BHI Broth | $10^5$ | $10^6$ | $10^7$ | $10^8$ |
| Microorganism CaptureEfficiency (%) | 68 | 57 | 51 | 72 |

**Examples 25 and 26: Testing of Concentration Device 4**

Bacterial suspensions (100 mL and 250 mL) of *Listeria innocua* in BHI Broth were prepared essentially according to the procedure of Example 18, each having a final concentration of $10^5$ CFUs/mL ($10^7$ CFUs total). A sterilized 48 diameter mm disk of the matrix from Example 4 was placed in the positive pressure manual filtration device, and the 100 mL bacterial suspension was plunged through the disk. The device was dissasembled to transfer the disk to a sterile culture dish for further analysis. The device was re-assembled with a clean disk, and the 250 mL bacterial suspension was plunged through it. The capacity of the manual filtration device was 100 mL, so the 250 mL bacterial suspension was plunged in amounts of 100 mL, 100 mL, and 50 mL. All of the suspensions passed through the disk, indicating that there was no plugging. The resulting filtrates, as well as unfiltered control suspensions, were diluted, plated, and incubated essentially according to the procedure of Example 18. Colonies were counted manually, and Microorganism Capture Efficiency was calculated. Results are shown in Table 5.

The post-filtration disks from the 100 mL and 250 mL filtration steps were cut apart using sterilized scissors and added to sterile 50 mL polypropylene centrifuge tubes containing 1 mL buffer solution for boiling. The disks were then processed according to the manufacturer's instructions in an ELISA assay and found to be positive (visual immunoassay using reference card of kit).

TABLE 5.

| Example No. | Concentration Device | Sample Volume (mL) | Total CFUs in Sample | CFUs in Filtrate | CFUs Captured by Disk | MCE (%) | Fold Concentration |
|---|---|---|---|---|---|---|---|
| 25 | 4 | 100 | $1.9 \times 10^7$ | $0.5 \times 10^7$ | $1.4 \times 10^7$ | 68 | 100X |
| 26 | 4 | 250 | $3.5 \times 10^7$ | $1 \times 10^7$ | $2.5 \times 10^7$ | 60 | 250X |

The data in Table 5 shows that the disks effectively concentrated the bacteria in the relatively large sample volumes that were utilized. The bacterial suspensions were concentrated from an initial 100 mL or 250 mL into a small amount (less than 1 mL) needed for an ELISA assay (see fold concentration in Table 5). Even when boiled in buffer, the disks did not disintegrate, and disk materials did not significantly interfere with the assay.

**Examples 27-29 and Comparative Example C6: Testing of Concentration Devices 4, 6, 7, and C3**

A 1000 mL flask, having a side vacuum port, was fitted with a sintered stopper that served as the support for a porous fibrous nonwoven matrix or filter. The support area was sized to hold a sterilized 36 mm or 48 mm diameter circular disk of the porous fibrous nonwoven matrix. An open-ended collection cylinder (100 mL capacity), having flanged rims around the top and bottom of the cylinder, was clamped to the flask with the stopper secured between them. A flexible hose connected the flask to a faucet equipped with a vacuum port to provide a vacuum filtration apparatus. The apparatus was sterilized by autoclaving at 121 °C for 15 minutes before each period of use and, during use, was rinsed with 70 weight percent (wt %) ethanol and distilled water after filtration of each sample.

Disks were die punched from the porous fibrous nonwoven matrices of Examples 4, 6, 7, and C3 and sterilized. The disks from Examples 4 and 6 were 48 mm in diameter, and the disks from Examples 7 and C3 were 36 mm in diameter.

Four 100 mL bacterial suspensions of *Listeria innocua* in BHI broth, each containing approximately $10^5$ CFUs/mL ($10^7$ CFUs total) were prepared essentially according to the procedure for Example 18. For each disk, a suspension was poured into the collection cylinder, and vacuum was applied.

The resulting filtrates, as well as pre-filtration suspensions, were diluted, plated, and incubated essentially according to the procedure of Example 18. Colonies were counted manually, and Microorganism Capture Efficiency was calculated. Results are shown in Table 6.

TABLE 6.

| Example No. | 27 | 28 | 29 | C6 |
|---|---|---|---|---|
| Concentration Device | 4 | 6 | 7 | C3 |
| Disk Thickness (mm) | 3 | 3 | 6 | 3 |
| MCE (%) | 78 | 77 | 89 | 10 |

Since vacuum filtration was utilized, filtration was effected relatively rapidly. Bacterial capture by the disks of Concentration Devices 4, 6, and 7 was nonetheless greater than 70%.

**Example 30 and Comparative Examples C7 and C8: Testing of Concentration Devices 9 and C3 and Comparison With Particulate Concentration Agent Alone (AS-Talc)**

A streak culture of *Listeria monocytogenes* (ATCC 51414) was used to prepare a 0.5 McFarland Standard (a turbidity standard comprising dispersed microorganisms) in 3 mL of BHI Broth using a DensiCHEK™ densitometer from bioMerieux, Inc., Durham, NC. The resulting bacterial stock, containing approximately $10^8$ CFUs/mL, was serially diluted in BHI broth to obtain a bacterial suspension having approximately $10^3$ CFUs/mL.

A 14 mm diameter disk was die punched from the matrix of Example 9, sterilized, and inserted into a filter holder (13 mm diameter Swinnex™ filter holder; Millipore Corp., Bedford, MA). A 3 cubic centimeter (cc) syringe was used to deliver 1.5 mL of the bacterial suspension onto the disk in the holder. The suspension was filtered manually, and filtration was completed in 20 seconds. A disk from Example C3 was also prepared, tested in the same manner, and filtered in the same amount of time.

The resulting filtrates were plated in 100 microliter volumes (undiluted) on MOX plates. The disks were removed from the filter holder after each test using surface-sterilized forceps and plated on MOX plates with 100 microliters of buffer solution. The plates were incubated at 37°C for 18-20 hours. Colonies were counted manually, and Microorganism Capture Efficiency was calculated. Results are shown in Table 7.

Example C5 was prepared by adding 20 mg AS-Talc powder to 1.1 mL of the bacterial suspension in a sterile 5 mL polypropylene tube (BD Falcon™ from VWR, West Chester, PA). The tube was capped and placed on a rocking platform (Thermolyne Vari Mix™ rocking platform from Barnstead International, Iowa) rocking at 14 cycles per minute for 20 seconds. Then the tube was transferred to a stand for 1 minute, after which time most of the particles of AS-Talc powder had settled to the bottom of the tube.

A volume of 100 microliters of the resulting supernatant (containing suspended AS-Talc particles) was plated on a MOX plate and processed (incubated) in essentially the same manner as the plates for the disks. A volume of 100 microliters of the bacterial suspension was diluted 1:10 and also plated and incubated in essentially the same manner as a control (pre-filtration sample). The colony count on the control was 2600 CFUs. Colonies were counted manually, and Microorganism Capture Efficiency was calculated. Results are shown in Table 7.

**TABLE 7.**

| Example No. | Concentration Device or Agent | Microorganism Capture Efficiency (%) |
| --- | --- | --- |
| C8 | AS-Talc Particles | 66 |
| C7 | Concentration Device C3 | 40 |
| 30 | Concentration Device 9 | 99 |

### Example 31 and Comparative Examples C9 and C10: Testing of Concentration Devices 2 and C1 and a Commercial Nylon Filter

Ground turkey (labeled 12% fat) was purchased from a local grocery store. 11 g of the ground turkey was placed in a sterile stomacher bag and blended with 99 mL buffer solution in a stomacher at a speed of 230 revolutions per minute (rpm) for 30 seconds. The blended sample was poured into the above-described positive pressure manual filtration device (see Example 18) containing a sterilized 48 mm disk of the matrix of Example 2 (for Example 31). Positive pressure was applied by pushing on the plunger of the device until 100 mL of blended sample passed through the disk, and the filtration time was recorded. The procedure was repeated with a disk from Example C1 (for Comparative Example C9) and with a 0.45 micron nylon filter (for Comparative Example C10) obtained from 3M Purification, Inc., St. Paul, MN. Results are shown in Table 8 below.

The procedure was repeated using pasteurized, pulp-free orange juice purchased from a local grocery store. A volume of 11 g of juice was added to 99 mL of buffer solution, swirled for about one minute to mix, and 100 mL of the resulting sample was filtered. The filtration time was measured, and results are shown in Table 8.

**TABLE 8.**

| Example No. | Concentration Device | Sample Volume Filtered (mL) | |
| --- | --- | --- | --- |
| | | Blended Turkey | Orange Juice |
| 31 | 2 | 15 | 12 |
| C9 | C1 | 26 | 14 |
| C10 | Commercial Nylon Filter | 2.5 | less than 1 |

The data in Table 8 show that meat and juice samples were successfully filtered through the disks of Example 31 and Comparative Example C9, which were somewhat more resistant to clogging than the standard microbiology filter of Comparative Example C10.

### Examples 32-43 and Comparative Examples C11-C13: Testing of Concentration Devices 6, 8-17, C3-C4, and a Commercial Polycarbonate Filter Membrane

Frozen ground beef (labeled 15% fat) was purchased from a local grocery store. 11 g of thawed ground beef was blended with 99 mL of buffer solution in a sterile stomacher bag and was processed in a stomacher at 230 rpm for 30 seconds. Disks of matrices from the Examples and Comparative Examples shown in Table 9 were tested essentially according to the procedure of Example 31. A commercial filter membrane (Whatman 14 mm diameter, 0.22 micron polycarbonate filter membrane purchased from VWR, West Chester, PA) was also tested as Comparative Example C13. The volume of blended beef passing through the disk or membrane prior to plugging and flow stoppage, and the time period of passage prior to plugging and flow stoppage, were recorded and are shown in Table 9.

A second series of the same matrices were tested using a soymilk purchased from a local grocery store. Samples were prepared by swirling 11 mL of soymilk with 99 mL of buffer solution. Results are shown in Table 9.

**TABLE 9.**

| Example No. | Concentration Device | Ground Beef | | Soymilk | |
|---|---|---|---|---|---|
| | | Volume (mL) | Time (seconds) | Volume (mL) | Time (seconds) |
| 33 | 8 | 10 | 8 | 10 | 20 |
| 32 | 6 | 1 | 20 | 1 | 15 |
| 34 | 9 | 8 | 60 | 0 | NA* |
| 35 | 10 | 10 | 100 | 0 | NA* |
| 36 | 11 | 10 | 60 | 1 | 30 |
| 37 | 12 | 6 | 30 | 1 | 15 |
| 38 | 13 | 4 | 20 | 1 | 15 |
| 39 | 14 | 2 | 20 | 1 | 15 |
| 40 | 15 | 6 | 36 | 4 | 25 |
| 41 | 16 | 9 | 10 | 10 | 80 |
| 42 | 17 | 5 | 30 | 1 | 40 |
| C11 | C3 | 2 | 20 | 2 | 25 |
| C12 | C4 | 4 | 20 | 1 | 15 |
| C13 | Commercial Polycarbonate Filter Membrane | 0.5 | 30 | 0 | NA* |
| *NA indicates that filtration was terminated after 30 seconds without noticeable flow through the concentration device. | | | | | |

## Example 43: Testing of Concentration Device 14

A streak culture of *Saccharomyces cerevisiae* (ATCC 201390) from a YPD agar plate, incubated at 30°C, was used to make a 0.5 McFarland Standard in 3 mL of beer. The beer was purchased from a local store. The resulting yeast stock, containing approximately $10^6$ CFUs/mL, was diluted serially in unspiked beer to obtain a yeast suspension containing $10^5$ CFUs/mL. A 1:100 dilution of the suspension was inoculated into 100 mL of beer to provide 10 CFUs/mL (total of approximately 1000 CFUs in the sample). The spiked beer was delivered to the above-described filter holder, containing a sterilized 14 mm disk die-punched from Example 14, in five batches using a 20 cc syringe. After the entire 100 mL sample passed through the disk, the filter holder was disassembled and the disk was transferred, using surface-sterilized forceps, to an empty sterile 1.5 mL polypropylene cuvette (3M™ CLEAN-TRACE™ Surface ATP sampling device; 3M Company, St. Paul, MN).

A Concentrated Sample was prepared by adding 100 microliters of the enzymatic system and 50 microliters of the extractant from the kit (3M™ CLEAN-TRACE™ Surface ATP system; 3M Company, St. Paul, MN) to the cuvette. The contents of the cuvette were mixed by vortexing for 5 seconds at about 3200 rpm on a vortex mixer (VWR™ Fixed Speed vortex mixer; VWR, West Chester, PA). The ATP signal of the Concentrated Sample was determined by measuring the relative light units (RLUs) for a minute at 10 second intervals using a bench-top luminometer (20/20n single tube luminometer from Turner Biosystems, Sunnyvale, CA). Luminescence values (ATP signal) were obtained from the luminometer using 20/20n SIS software that was provided with the luminometer. Results are shown in Table 10.

The ATP signal was also determined according to the above procedure for controls using 100 microliters of the yeast suspension containing $10^3$ CFUs ($10^3$ CFU Control) from a 1:10 dilution of the $10^4$ CFUs/mL yeast stock (100% signal control) and using 100 microliters of the unfiltered spiked beer samples (Spiked Beer Control). The background ATP levels were determined on a sample prepared by filtering 100 mL of unspiked beer through the porous fibrous nonwoven matrix of Example 14 to obtain an Unspiked Filtrate, and processing the used matrix according to the procedure described above to obtain a Control Matrix. A 100 microliter volume of only the beer (without filtering) was also tested (Unspiked Beer Control). Results are shown in Table 10.

The % ATP signal, shown in Table 10, was calculated (based on the RLU values obtained from the Concentrated Sample after subtracting the background ATP signal from the Unspiked Beer Control (Corrected RLUs) and the RLUs from the $10^3$ CFU Control) as follows:

$$\% \text{ ATP} = (\text{Corrected RLUs } / \text{RLUs from } 10^3 \text{ CFU Control}) \times 100$$

Yeast counts were determined by plating 1 mL of the 100 mL beer samples (Spiked Beer Control, as well as a 1:100 diluted sample of $10^4$ CFUs/mL yeast stock) on Y/M plates according to the manufacturer's instructions. The diluted sample had a total of 1060 CFUs of yeast cells.

**TABLE 10.**

| Sample | ATP Signal (RLUs) | Corrected ATP Signal (RLUs) | % ATP Signal Relative to $10^3$ CFU Control |
|---|---|---|---|
| **Unspiked Beer Control** | 3352 | | |
| **$10^3$ CFUs Control** | 6479 | 3127 | 100% |
| **Spiked Beer Control** | 3592 | 240 | 8% |
| **Unspiked Filtrate Control** | 2815 | | |
| **Concentrated Sample** | 4776 | 1961 | 63% |

The data shows that the ATP signal of *S. cerevisiae* from the Concentrated Sample was approximately 8 times that of the unfiltered Spiked Beer Control.

**Examples 44-45 and Comparative Example C14: Testing of Concentration Devices 12, 13, and C4**

A streak culture of *Listeria monocytogenes* was used to make a 0.5 McFarland Standard in 3 mL of BHI Broth. The resulting bacterial stock, containing $10^8$ CFUs/mL, was serially diluted in BHI to provide a bacterial suspension containing approximately $10^3$ CFUs/mL.

A 14 mm disk was die-punched from the matrix of Example 12, inserted into the filter holder, and tested essentially according to the procedure described in Example 30. A volume of 1.5 mL of the suspension passed completely through the disk in 15 seconds. The filtration was repeated using disks from Example 13 and Comparative Example C4. The resulting filtrates from each disk, as well as the unfiltered bacterial suspension (control), were plated in 100 microliter volumes on MOX agar plates and incubated at 37°C for 18 - 20 hours. Colonies were counted manually, and Microorganism Capture Efficiency was determined. The unfiltered control sample had 2800 CFUs/mL. Results are shown in Table 11.

The used disk from each test was removed from the filter holder using surface-sterilized forceps and plated on MOX plates with 100 microliters of buffer solution. The plates were incubated at 37°c for 18-20 hours. All of the plates showed growth of *Listeria monocytogenes.*

**TABLE 11.**

| Example No. | Concentration Device | Microorganism Capture Efficiency (%) |
|---|---|---|
| **44** | 12 | 91 |
| **45** | 13 | 93 |
| **C14** | C4 | 75 |

**Examples 46-49 : Testing of Concentration Devices 8 and 12**

A streak culture of *Pseudomonas aeruginosa* (ATCC 9027) was used to make a 0.5 McFarland Standard in 3 mL of filtered distilled deionized water (18 megaohm water from a Milli-Q™. Gradient deionization system, Millipore, Bedford, MA). The resulting bacterial stock containing $10^8$ CFUs/mL was serially diluted in the same water to provide a $10^2$ CFUs/mL bacterial suspension in water.

A 14 mm disk of the porous fibrous nonwoven matrix of Example 8 (thickness of 1.066 mm) was die-punched and inserted into the above-described filter holder. A volume of 1 mL of the bacterial suspension was filtered manually essentially according to the procedure described in Example 30. Filtration was completed in 15 seconds. The resulting filtrates were plated on AC plates according to the manufacturer's instructions. The disk was removed from the filter holder using surface-sterilized forceps and was plated on PIA plates with 100 microliters of buffer solution.

The procedure was repeated using a disk of the matrix from Example 12 (thickness of 1.336 mm). A 1 mL volume of the bacterial suspension in water was also plated on AC plates as a control. All of the plates were incubated at 37°C for 18-20 hours. Colony counts from plated filtrates on AC plates were obtained per manufacturer's instructions. The unfiltered bacterial suspension had a colony count of 140 CFUs/mL. The Microorganism Capture Efficiency results are shown in Table 12.

A *Staphylococcus aureus* (ATCC 6538) bacterial suspension was prepared in essentially the same manner from a streak culture of *S. aureus*. The suspension was filtered through disks that had been die-cut from the matrices of Examples 8 and 12, and the resulting filtrates were analyzed for Microorganism Capture Efficiency essentially according to the procedure described for *P. aeruginosa.* The unfiltered bacterial suspension had a colony count of 170 CFUs/mL. Results are shown in Table 12. The used disks were plated on C-agar plates and processed essentially according to the procedure used for *P. aeruginosa.*

**TABLE 12.**

| Example No. | Concentration Device | Microorganism | Microorganism Capture Efficiency (%) |
|---|---|---|---|
| 46 | 8 | *P. aeruginosa* | 59 |
| 47 | 12 | *P. aeruginosa* | 100 |
| 48 | 8 | *S. aureus* | 51 |
| 49 | 12 | *S. aureus* | 100 |

All of the PIA plates (containing disks through which bacterial suspensions of *P. aeruginosa* had passed) exhibited a yellow-green pigment, characteristic of the presence of *P. aeruginosa.* All of the C-agar plates (containing disks through which bacterial suspensions of *S. aureus* had passed) exhibited an orange-magenta color, characteristic of the presence of *S. aureus.*

**Example 50** - **Water Filtration Examples**

A streak culture of E coli (ATCC 51813) on a Blood Agar plate (Tryptic Soy Agar with 5% sheep's blood, Hardy Diagnostics, Santa Maria, CA) was incubated overnight at 37°C. The culture was used to prepare a 0.5 McFarland Standard using DensiCHEK™ densitometer (bioMerieux, Inc., Durham, NC) in 3 ml Butterfield's Buffer. The resulting bacterial stock containing 1x $10^8$ CFUs/mL was serially diluted in Butterfield's to obtain an inoculum with approximately 1 X $10^6$ CFU/mL.

A test sample was prepared by inoculating 100 ml deionized of water (MilliQ Gradient system, Millipore, Ma) a 1:100 dilution of the $10^6$ bacteria/ml inoculum resulting in water test sample containing $10^4$ CFU/ml ($10^6$ CFUs total in the water).

The inoculated water sample was pumped through a filtration device holding a 47 mm diameter die cut disk of the fibrous nonwoven matrix shown in Table 13. The device had a polycarbonate cylindrical body measuring about 60 mm in diameter and about 115 mm high and having a support screen to hold the filter disk in the body. The top end of the body was closed with a threaded cap having an inlet port attached to a peristaltic pump (Model No. 7553-70; Cole Parmer) by 1/8" thick wall PVC tubing (Catalog # 60985-522; VWR; Batavia, IL). The pump was used to deliver the water sample to the filtration device. The bottom end had an outlet port and was threaded to close the bottom of the cylinder. O-rings were positioned between the threaded parts to prevent leakage. The device was vented on the upstream side to allow for purging of air.

Each matrix was tested in duplicates. A 100 mL sample of inoculated water was pumped into the filtration device at a flow rate of 70 ml/minute. Filtrates were collected in sterile 100 ml polypropylene beakers. After each filtration test, the device was disassembled and the disk was removed using sterile forceps. Between each test, the filtration device was rinsed with 500 mL of filtered sterilized deionized water.

One hundred microliter volumes of each filtrate and a pre-filtration suspension, were diluted 1:10 and 1:100 in Butterfield's Buffer and plated on E.coli Plate. The plates were incubated at 37°C for 18-20 hours. Colony counts were determined from plates according to the manufacturer's instructions. The Log Reduction Value (LRV) is an indication of bacterial removal capacity of a water filter. The values were calculated based on counts obtained from the plated filtrate and pre-filtration samples by using the formula below:

$$LRV = (\text{Log of CFUs/ml in pre-filtration sample}) - (\text{Log of CFUs/ml in filtrate sample})$$

The pre-filtration suspension contained an average of 9500 CFU/ml (~4 Log CFU/ml).

Log reduction values are shown in Table 13.

**TABLE 13.**

| Example | Disk | LRV |
|---------|------------|-----|
| 50 | Example 7 | 3 |
| 51 | Example 10 | 4 |
| 52 | Example 11 | 4 |
| 53 | Example 13 | 4 |
| 54 | Example 16 | 4 |

**Claims**

1. A concentration process comprising

   (a) providing a concentration device comprising

   (1) a porous fibrous nonwoven matrix, and
   (2) a plurality of particles of at least one concentration agent that comprises an amorphous metal silicate having a surface composition having a metal atom to silicon atom ratio of less than or equal to 0.5, as determined by X-ray photoelectron spectroscopy (XPS), said particles being enmeshed in said porous fibrous nonwoven matrix;

   (b) providing a sample comprising at least one target cellular analyte, wherein the target cellular analyte is any cell or micro-organism suitable for analysis or detection;
   (c) contacting said concentration device with said sample such that at least a portion of said at least one target cellular analyte is bound to or captured by said concentration device; and
   (d) detecting the presence of at least one bound target cellular analyte.

2. The process of Claim 1, wherein said porous fibrous nonwoven matrix is formed by a wetlaid process.

3. The process of Claim 1 or Claim 2, wherein said porous fibrous nonwoven matrix comprises at least one fibrillated fiber.

4. The process of Claim 1 or any other of the preceding claims, wherein the fibers of said porous fibrous nonwoven matrix are selected from combinations of polymer fibers and inorganic fibers.

5. The process of Claim 4, wherein said polymer fibers comprise at least one polymer selected from polyamides, polyolefins, polysulfones, and combinations thereof.

6. The process of Claim 4 or Claim 5, wherein said inorganic fibers comprise at least one inorganic material selected from glasses, ceramics, and combinations thereof.

7. The process of Claim 1 or any other of the preceding claims, wherein said porous fibrous nonwoven matrix comprises at least one polymeric binder.

8. The process of Claim 1 or any other of the preceding claims, wherein said particles of at least one concentration agent comprise microparticles.

9. The process of Claim 1 or any other of the preceding claims, wherein said metal silicate is amorphous.

10. The process of Claim 1 or any other of the preceding claims, wherein said metal is selected from magnesium, calcium, zinc, aluminum, iron, titanium, and combinations thereof.

11. A concentration device adapted for binding or capturing at least one target cellular analyte,

wherein the target cellular analyte is any cell or micro-organism
suitable for analysis or detection, the concentration device comprising

(a) a porous fibrous nonwoven matrix comprising polymer fibers and inorganic fibers; and
(b) a plurality of particles of at least one concentration agent that comprises an amorphous, spheroidized metal silicate having a surface composition having a metal atom to silicon atom ratio of less than or equal to 0.5, as determined by X-ray photoelectron spectroscopy (XPS); wherein said particles are enmeshed in said porous fibrous nonwoven matrix.

12. A kit comprising

(a) at least one concentration device of Claim 11; and
(b) at least one testing container or testing reagent for use in carrying out the process of Claim 1.

13. A process for preparing a concentration device adapted for binding or capturing at least one target cellular analyte, wherein the target cellular analyte is any cell or micro-organism suitable for analysis or detection, the concentration device comprising

(a) providing a plurality of fibers comprising polymer fibers and inorganic fibers;
(b) providing a plurality of particles of at least one concentration agent that comprises an amorphous, spheroidized metal silicate having a surface composition having a metal atom to silicon atom ratio of less than or equal to 0.5, as determined by X-ray photoelectron spectroscopy (XPS); and
(c) forming at least a portion of said plurality of fibers into a porous fibrous nonwoven matrix having at least a portion of said plurality of particles enmeshed therein.

14. The process of Claim 13, wherein said forming is carried out by a wetlaid process;
and/or wherein said amorphous, spheroidized metal silicate is amorphous, spheroidized magnesium silicate.

**Patentansprüche**

1. Konzentrationsverfahren, umfassend

(a) Bereitstellen einer Konzentrationsvorrichtung, umfassend

(1) eine poröse Faservliesmatrix und
(2) eine Mehrzahl von Teilchen von mindestens einem Konzentrationsmittel, das ein amorphes Metallsilikat mit einer Oberflächenzusammensetzung, die ein Metallatom-zu-Siliciumatom-Verhältnis von weniger als oder gleich 0,5 aufweist, umfasst, wie durch Röntgenphotoelektronenspektroskopie (XPS) bestimmt, wobei die Teilchen in der porösen Faservliesmatrix eingebunden sind;

(b) Bereitstellen einer Probe, umfassend mindestens einen zellulären Zielanalyten, wobei der zelluläre Zielanalyt jede Zelle oder jeder Mikroorganismus ist, der zur Analyse oder zum Nachweis geeignet ist;
(c) Inkontaktbringen der Konzentrationsvorrichtung mit der Probe, sodass mindestens ein Teil des mindestens einen zellulären Zielanalyten an die Konzentrationsvorrichtung gebunden oder von dieser eingefangen wird; und

(d) Detektieren des Vorhandenseins von mindestens einem gebundenen zellulären Zielanalyten.

2. Verfahren nach Anspruch 1, wobei die poröse Faservliesmatrix durch ein Nasslegeverfahren gebildet wird.

3. Verfahren nach Anspruch 1 oder Anspruch 2, wobei die poröse Faservliesmatrix mindestens eine fibrillierte Faser umfasst.

4. Verfahren von Anspruch 1 oder einem anderen der vorstehenden Ansprüche, wobei die Fasern der porösen Faservliesmatrix ausgewählt sind aus Kombinationen von polymeren Fasern und anorganischen Fasern.

5. Verfahren nach Anspruch 4, wobei die polymeren Fasern mindestens ein Polymer umfassen, das ausgewählt ist aus Polyamiden, Polyolefinen, Polysulfonen und Kombinationen davon.

6. Verfahren nach Anspruch 4 oder Anspruch 5, wobei die anorganischen Fasern mindestens ein anorganisches Material umfassen, das ausgewählt ist aus Gläsern, Keramiken und Kombinationen davon.

7. Verfahren nach Anspruch 1 oder einem anderen der vorstehenden Ansprüche, wobei die poröse Faservliesmatrix mindestens ein polymeres Bindemittel umfasst.

8. Verfahren nach Anspruch 1 oder einem anderen der vorstehenden Ansprüche, wobei die Teilchen von mindestens einem Konzentrationsmittel Mikroteilchen umfassen.

9. Verfahren nach Anspruch 1 oder einem anderen der vorstehenden Ansprüche, wobei das Metallsilikat amorph ist.

10. Verfahren nach Anspruch 1 oder einem anderen der vorstehenden Ansprüche, wobei das Metall ausgewählt ist aus Magnesium, Calcium, Zink, Aluminium, Eisen, Titan und Kombinationen davon.

11. Konzentrationsvorrichtung, angepasst
zum Binden oder Einfangen mindestens eines zellulären Zielanalyten, wobei der zelluläre Zielanalyt jede Zelle oder jeder Mikroorganismus ist, der zur Analyse oder zum Nachweis geeignet ist, die Konzentrationsvorrichtung umfassend

(a) eine poröse Faservliesmatrix, umfassend polymere Fasern und anorganische Fasern; und
(b) eine Mehrzahl von Teilchen von mindestens einem Konzentrationsmittel, das ein amorphes sphäroidisiertes Metallsilikat mit einer Oberflächenzusammensetzung, die ein Metallatom-zu-Siliciumatom-Verhältnis von weniger als oder gleich 0,5 aufweist, umfasst, wie durch Röntgenphotoelektronenspektroskopie (XPS) bestimmt; wobei die Teilchen in der porösen Faservliesmatrix eingebunden sind.

12. Kit, umfassend

(a) mindestens eine Konzentrationsvorrichtung nach Anspruch 11; und
(b) mindestens einen Testbehälter oder ein Testreagenz zur Verwendung beim Ausführen des Verfahrens nach Anspruch 1.

13. Verfahren zum Herstellen einer Konzentrationsvorrichtung, angepasst zum Binden oder Einfangen von mindestens einem zellulären Zielanalyten, wobei der zelluläre Zielanalyt jede Zelle oder jeder Mikroorganismus ist, der zur Analyse oder zum Nachweis geeignet ist, die Konzentrationsvorrichtung umfassend

(a) Bereitstellen einer Mehrzahl von Fasern, die polymere Fasern und anorganische Fasern umfasst;
(b) Bereitstellen einer Mehrzahl von Teilchen von mindestens einem Konzentrationsmittel, das ein amorphes sphäroidisiertes Metallsilikat mit einer Oberflächenzusammensetzung, die ein Metallatom-zu-Siliciumatom-Verhältnis von weniger als oder gleich 0,5 aufweist, umfasst, wie durch Röntgenphotoelektronenspektroskopie (XPS) bestimmt; und
(c) Bilden mindestens eines Teils der Mehrzahl von Fasern zu einer porösen Faservliesmatrix mit mindestens einem Teil der Mehrzahl von Teilchen darin eingebunden.

14. Verfahren nach Anspruch 13, wobei das Bilden durch ein Nasslegeverfahren ausgeführt wird; und/oder wobei das amorphe sphäroidisierte Metallsilikat amorphes sphäroidisiertes Magnesiumsilikat ist.

**Revendications**

1. Procédé de concentration comprenant

    (a) la fourniture d'un dispositif de concentration comprenant

        (1) une matrice non tissée fibreuse poreuse, et
        (2) une pluralité de particules d'au moins un agent de concentration qui comprend un silicate de métal amorphe ayant une composition de surface ayant un rapport d'atome métallique à atome de silicium inférieur ou égal à 0,5, tel que déterminé par spectrométrie photoélectronique à rayons X (XPS), lesdites particules étant enchevêtrées dans ladite matrice non tissée fibreuse poreuse ;

    (b) la fourniture d'un échantillon comprenant au moins un analyte cellulaire cible, dans lequel l'analyte cellulaire cible est n'importe quelle cellule ou n'importe quel micro-organisme approprié pour analyse ou détection ;
    (c) la mise en contact dudit dispositif de concentration avec ledit échantillon de telle sorte qu'au moins une partie dudit au moins un analyte cellulaire cible est liée à ou capturée par ledit dispositif de concentration ; et
    (d) la détection de la présence d'au moins un analyte cellulaire cible lié.

2. Procédé selon la revendication 1, dans lequel ladite matrice non tissée fibreuse poreuse est formée par un procédé appliqué par voie humide.

3. Procédé selon la revendication 1 ou la revendication 2, dans lequel ladite matrice non tissée fibreuse poreuse comprend au moins une fibre fibrillaire.

4. Procédé selon la revendication 1 ou l'une quelconque des revendications précédentes, dans lequel les fibres de ladite matrice non tissée fibreuse poreuse sont choisies parmi des combinaisons de fibres polymères et de fibres inorganiques.

5. Procédé selon la revendication 4, dans lequel lesdites fibres polymères comprennent au moins un polymère choisi parmi polyamides, polyoléfines, polysulfones, et combinaisons de ceux-ci.

6. Procédé selon la revendication 4 ou la revendication 5, dans lequel lesditesfibres inorganiques comprennent au moins un matériau inorganique choisi parmi verres, céramiques, et combinaisons de ceux-ci.

7. Procédé selon la revendication 1 ou l'une quelconque des revendications précédentes, dans lequel ladite matrice non tissée fibreuse poreuse comprend au moins un liant polymère.

8. Procédé selon la revendication 1 ou l'une quelconque des revendications précédentes, dans lequel lesdites particules d'au moins un agent de concentration comprennent des microparticules.

9. Procédé selon la revendication 1 ou l'une quelconque des revendications précédentes, dans lequel ledit silicate de métal est amorphe.

10. Procédé selon la revendication 1 ou l'une quelconque des revendications précédentes, dans lequel ledit métal est choisi parmi magnésium, calcium, zinc, aluminium, fer, titane, et combinaisons de ceux-ci.

11. Dispositif de concentration conçu pour lier ou capturer au moins un analyte cellulaire cible,
    dans lequel l'analyte cellulaire cible est n'importe quelle cellule ou n'importe quel micro-organisme approprié pour analyse ou détection, le dispositif de concentration comprenant

    (a) une matrice non tissée fibreuse poreuse comprenant des fibres polymères et des fibres inorganiques ; et
    (b) une pluralité de particules d'au moins un agent de concentration qui comprend un silicate de métal amorphe sphéroïdisé ayant une composition de surface ayant un rapport d'atome métallique à atome de silicium inférieur ou égal à 0,5, tel que déterminé par spectrométrie photoélectronique à rayons X (XPS) ;

    dans lequel lesdites particules sont enchevêtrées dans ladite matrice non tissée fibreuse poreuse.

12. Trousse comprenant

(a) au moins un dispositif de concentration selon la revendication 11 ; et
(b) au moins un récipient d'essai ou réactif d'essai pour utilisation dans la réalisation du procédé selon la revendication 1.

13. Procédé de préparation d'un dispositif de concentration conçu pour lier ou capturer au moins un analyte cellulaire cible, dans lequel l'analyte cellulaire cible est n'importe quelle cellule ou n'importe quel micro-organisme approprié pour analyse ou détection, le dispositif de concentration comprenant

(a) la fourniture d'une pluralité de fibres comprenant des fibres polymères et des fibres inorganiques ;
(b) la fourniture d'une pluralité de particules d'au moins un agent de concentration qui comprend un silicate de métal amorphe sphéroïdisé ayant une composition de surface ayant un rapport d'atome métallique à atome de silicium inférieur ou égal à 0,5, tel que déterminé par spectrométrie photoélectronique à rayons X (XPS) ; et
(c) la formation d'au moins une partie de ladite pluralité de fibres en une matrice non tissée fibreuse poreuse ayant au moins une partie de ladite pluralité de particules enchevêtrées en son sein.

14. Procédé selon la revendication 13, dans lequel ladite formation est effectuée par un procédé appliqué par voie humide ; et/ou dans lequel ledit silicate de métal amorphe, sphéroïdisé est du silicate de magnésium amorphe sphéroïdisé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9306924 A
- WO 9802224 A
- WO 2010114727 A
- US 6045913 A
- US 20100190171 A, Kshirsagar
- US 61289213
- US 7422868 B, Fan
- WO 2008150779 A

**Non-patent literature cited in the description**

- 3M™ Cosmetic Microspheres CM-111. 3M Company